(19) Europäisches Patentamt
European Patent Office
Office européen des brevets

(11) Veröffentlichungsnummer: **0 271 780**
**A2**

# (12) EUROPÄISCHE PATENTANMELDUNG

(21) Anmeldenummer: **87117929.7**

(22) Anmeldetag: **04.12.87**

(51) Int. Cl.⁴: **C07D 239/42**, C07D 239/52, C07D 239/34, C07D 253/06, C07D 251/22, C07D 251/16, C07D 251/46, A01N 47/36, C07C 143/828, C07C 143/78, C07C 143/70

(30) Priorität: **16.12.86 DE 3642824**

(43) Veröffentlichungstag der Anmeldung:
**22.06.88 Patentblatt 88/25**

(84) Benannte Vertragsstaaten:
**AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Müller, Klaus-Helmut, Dr.**
**Bockhackstrasse 55**
**D-4000 Düsseldorf 13(DE)**
Erfinder: **Pfister, Theodor, Dr.**
**Lichtenberger Strasse 30**
**D-4019 Monheim(DE)**
Erfinder: **Riebel, Hans-Jochem, Dr.**
**In der Beek 92**
**D-5600 Wuppertal 1(DE)**
Erfinder: **Kysela, Ernst, Dr.**
**Virchowstrasse 14**
**D-5060 Bergisch-Gladbach 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Robert Harry, Dr.**
**Unterdorfstrasse 6A**
**D-4000 Düsseldorf 31(DE)**

(54) **Substituierte 1-Benzylsulfonyl-3-heteroaryl-(thio)harnstoffe.**

(57) Die Erfindung betrifft neue substituierte 1-Benzylsulfonyl-3-heteroaryl-(thio)harnstoffe der allgemeinen Formel (I)

EP 0 271 780 A2

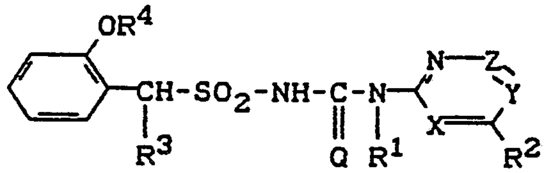

(I)

(worin Q, $R^1$, $R^2$, $R^3$, $R^4$, X, Y und Z die in der Beschreibung angegebenen Bedeutungen haben) sowie Salze von Verbindungen der Formel (I) mit Metallen und basischen organischen Stickstoffverbindungen, ferner Verfahren und neue Zwischenprodukte zu ihrer Herstellung und ihre Verwendung als Herbizide.

## Substituierte 1-Benzylsulfonyl-3-heteroaryl-(thio)harnstoffe

Die Erfindung betrifft neue substituierte 1-Benzylsulfonyl-3-heteroaryl-(thio)harnstoffe, ein Verfahren und neue Zwischenprodukte zu ihrer Herstellung sowie ihre Verwendung als Herbizide.

Es ist bekannt, daß bestimmte 1-Benzylsulfonyl-3-heteroaryl-(thio)harnstoffe, wie z. B. 1-(2-Methoxycarbonylbenzylsulfonyl)-3-(4,6-dimethoxy-pyrimidin-2-yl)-harnstoff, herbizid wirksam sind. Die Wirkung dieser Verbindungen ist jedoch nicht immer ganz befriedigend (vgl. US-PS 4 420 325).

Es wurden nun neue substituierte 1-Benzylsulfonyl-3-heteroaryl-(thio)harnstoffe der allgemeinen Formel (I)

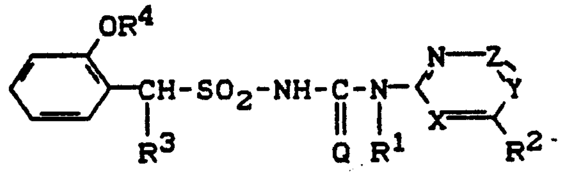

in welcher

Q für Sauerstoff oder Schwefel steht,

$R^1$ für Wasserstoff oder für gegebenenfalls substituierte Reste aus der Reihe Alkyl, Alkenyl, Alkinyl oder Aralkyl steht,

$R^2$ für Wasserstoff, Halogen, Hydroxy, Alkyl, Halogenalkyl, Alkoxy, Halogenalkoxy, Alkylthio, Halogenalkylthio, Amino, Alkylamino oder Dialkylamino steht,

$R^3$ für Wasserstoff oder Alkyl steht,

$R^4$ für Halogenalkyl steht,

X für Stickstoff oder eine -CH-Gruppierung steht,

Y für Stickstoff oder eine -$CR^5$-Gruppierung steht, worin

$R^5$ für Wasserstoff, Halogen, Cyano, Alkyl, Formyl, Alkyl-carbonyl oder Alkoxy-carbonyl steht und

Z für Stickstoff oder eine -$CR^6$-Gruppierung steht, worin

$R^6$ für Wasserstoff, Halogen, Hydroxy, Alkyl, Halogenalkyl, Alkoxy, Halogenalkoxy, Alkylthio, Alkylamino oder Dialkylamino steht,

sowie Salze von Verbindungen der Formel (I) mit Metallen und basischen organischen Stickstoffverbindungen gefunden.

Man erhält die neuen substituierten 1-Benzylsulfonyl-3-heteroaryl-(thio)harnstoffe der Formel (I), wenn man 2-Halogenalkoxy-benzylsulfonyl-iso(thio)cyanate der Formel (II)

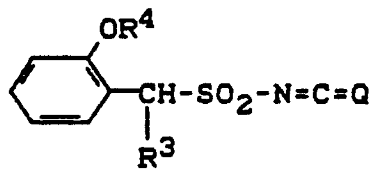

(II)

in welcher

Q, $R^3$ und $R^4$ die oben angegebenen Bedeutungen haben,

mit Heteroarylaminen der Formel (III)

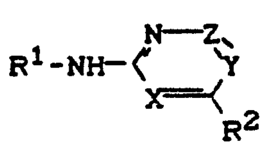

(III)

in welcher

$R^1$, $R^2$, X, Y und Z die oben angegebenen Bedeutungen haben,

gegebenenfalls in Gegenwart von Verdünnungsmitteln und gegebenenfalls in Gegenwart von Katalysatoren umsetzt und gegebenenfalls die so erhaltenen Produkte nach üblichen Methoden in Salze überführt.

Die neuen substituierten 1-Benzylsulfonyl-3-heteroaryl-(thio)harnstoffe der Formel (I) zeichnen sich durch starke herbizide Wirksamkeit aus.

Überraschenderweise zeigen die neuen Verbindungen der Formel (I) erheblich bessere herbizide Wirkung als vorbekannte 1-Benzylsulfonyl-3-heteroaryl-(thio)harnstoffe gleicher Wirkungsrichtung.

Weitere mögliche Herstellungsmethoden für die erfindungsgemäßen Verbindungen der Formel (I) sind nachstehend aufgeführt, wobei Q, $R^1$, $R^2$, $R^3$, $R^4$, X, Y und Z die oben angegebenen Bedeutungen haben:

(a) Umsetzung von 2-Halogenalkoxy-benzylsulfonamiden (IV) mit N-Heteroaryl-urethanen (V), ($R^7$ : Alkyl, Benzyl, Phenyl):

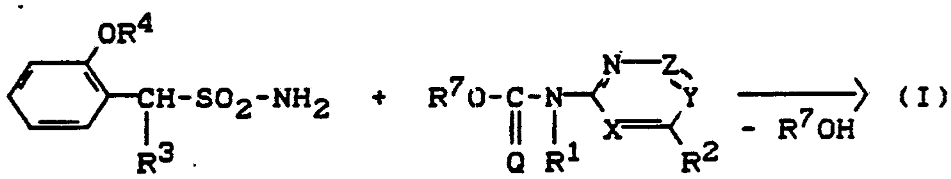

(IV) (V)

(b) Umsetzung von N-(2-Halogenalkoxy-benzylsulfonyl)urethanen (VI) ($R^8$ : Alkyl, Benzyl, Phenyl) mit Heteroarylaminen (III):

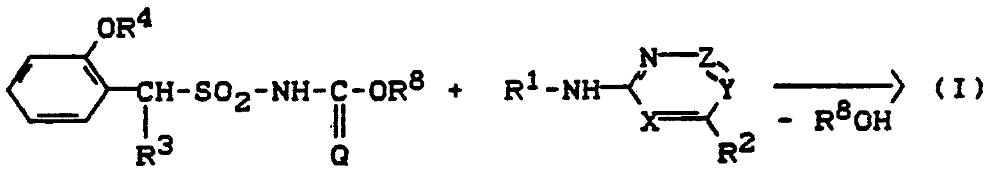

(VI) (III)

(c) Umsetzung von 1-Benzylsulfonyl-3-heteroaryl-(thio)harnstoffen (VII) mit "Alkylierungsmitteln" (VIII) (W : nucleophile Abgangsgruppe):

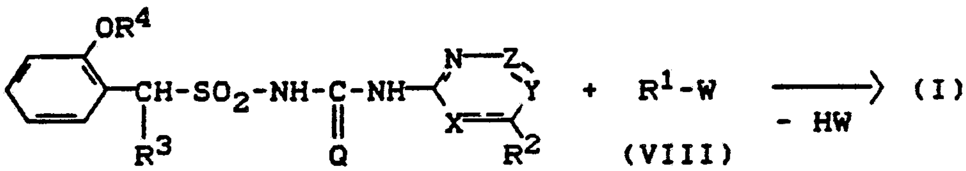

(VII) (VIII)

Gegenstand der Erfindung sind vorzugsweise Verbindungen der Formel (I), in welcher

Q für Sauerstoff oder Schwefel steht,

$R^1$ für Wasserstoff oder für $C_1$-$C_6$-Alkyl [welches gegebenenfalls durch Fluor, Cyano, $C_1$-$C_4$-Alkoxy oder $C_1$-$C_4$-Alkylthio substituiert ist], für $C_3$-$C_6$-Alkenyl und $C_3$-$C_6$-Alkinyl [welche gegebenenfalls durch Fluor oder Chlor substituiert sind] oder für Phenyl-$C_1$-$C_2$-alkyl [welches im Phenylteil gegebenenfalls durch Fluor, Chlor, Nitro, Cyano, Methyl, Methoxy oder $C_1$-$C_2$-Alkoxycarbonyl substituiert ist] steht,

$R^2$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethyl amino, Dimethylamino oder Diethylamino steht,

$R^3$ für Wasserstoff oder $C_1$-$C_4$-Alkyl steht,

$R^4$ für Halogen-$C_1$-$C_4$-alkyl steht, wobei Halogen für Fluor und/oder Chlor steht,

X für Stickstoff oder eine -CH-Gruppierung steht,

Y für Stickstoff oder eine -$CR^5$-Gruppierung steht,

worin

$R^5$ für Wasserstoff, Fluor, Chlor, Brom, Methyl, Formyl, Acetyl, Methoxycarbonyl oder Ethoxycarbonyl steht und

Z für Stickstoff oder eine -$CR^6$-Gruppierung steht,

worin

$R^6$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

Gegenstand der Erfindung sind weiterhin vorzugsweise Salze von Verbindungen der Formel (I) - wie vorausgehend definiert - mit Natrium, Kalium, Magnesium, Calcium sowie mit Monoalkyl-, Dialkyl-, Trialkyl-, Benzyl-alkyl-und Benzyl-dialkylamine mit jeweils bis zu 4 Kohlenstoffatomen in den Alkylresten, wobei die Alkylreste gegebenenfalls jeweils einen Substituenten aus der Reihe Fluor, Chlor, Hydroxy, Methoxy, Ethoxy oder Cyano enthalten.

Gegenstand der Erfindung sind insbesondere Verbindungen der Formel (I), in welcher
Q für Sauerstoff steht,
$R^1$ für Wasserstoff oder Methyl steht,
$R^2$ für Wasserstoff, Chlor, Methyl, Ethyl, Trifluormethyl, Methoxy, Ethoxy oder Difluormethoxy steht,
$R^3$ für Wasserstoff steht,
$R^4$ für Halogen-$C_1$-$C_2$-alkyl steht, wobei Halogen für Fluor und/oder Chlor steht,
X für Stickstoff oder eine -CH-Gruppierung steht,
Y für Stickstoff oder eine -$CR^5$-Gruppierung steht,
worin
$R^5$ für Wasserstoff, Fluor, Chlor oder Methyl steht und
Z für Stickstoff oder eine -$CR^6$-Gruppierung steht,
worin
$R^6$ für Wasserstoff, Chlor, Methyl, Ethyl, Trifluormethyl, Methoxy, Ethoxy oder Difluormethoxy steht.

Verwendet man beispielsweise für das erfindungsgemäße Verfahren 2-Trifluormethoxy-benzylsulfonylisocyanat und 2-Amino-4,6-dimethyl-pyrimidin als Ausgangsstoffe, so kann der Reaktionsablauf durch folgendes Formelschema skizziert werden:

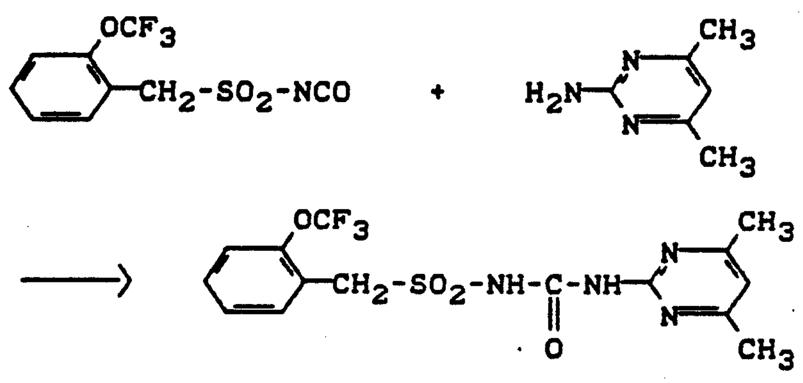

Die beim erfindungsgemäßen Verfahren als Ausgangsstoffe zu verwendenden 2-Halogenalkoxy-benzylsulfonyl-iso-(thio)cyanate sind durch die Formel (II) definiert. In dieser Formel stehen Q, $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Als Beispiele für die Ausgangsstoffe der Formel (II) seien genannt:
2-Difluormethoxy-, 2-Trifluormethoxy-, 2-Chlordifluormethoxy-, 2-Dichlorfluormethoxy-, 2-(2-Chlor-ethoxy-, 2-(2,2,2-Trifluorethoxy)-, 2-(1,1,2,2-Tetrafluor-ethoxy)-und 2-(2-Chlor-1,1,2-trifluor-ethoxy)-benzylsulfonylisocyanat bzw. -benzylsulfonylisothiocyanat, sowie 2-Difluormethoxy-, 2-Trifluormethoxy-, 2-Chlordifluor methoxy-, 2-Dichlorfluormethoxy, 2-(2-Chlor-ethoxy)-, 2-(1,1,2,2-Tetrafluor-ethoxy)-und 2-(2-Chlor-1,1,2-trifluor-ethoxy)-α-methyl-benzylsulfonylisocyanat bzw. -α-methyl-benzylsulfonyl-isothiocyanat.

Die Ausgangsverbindungen der Formel (II) sind noch nicht in der Literatur beschrieben. Man erhält diese Verbindungen, wenn man

(a) für den Fall, daß Q für Sauerstoff steht, 2-Halogenalkoxy-benzylsulfonamide der Formel (IV)

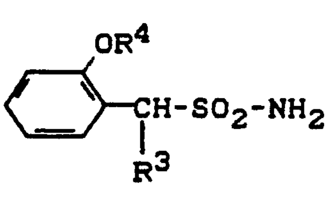

(IV)

in welcher

$R^3$ und $R^4$ die oben angegebenen Bedeutungen haben, mit etwa der äquimolaren Menge eines Alkylisocyanats, wie z. B. Butylisocyanat, gegebenenfalls in Gegenwart eines Katalysators, wie z. B. Diazabicyclo-[2,2,2]-octan (DABCO) und in Gegenwart eines Verdünnungsmittels, wie z. B. Xylol, bei Temperaturen zwischen 50 °C und 200 °C, vorzugsweise zweischen 80 °C und 150 °C, umsetzt und dabei mindestens die äquimolare Menge Phosgen einleitet, oder

(b) für den Fall, daß Q für Schwefel steht, wenn man 2-Halogenalkoxy-benzylsulfonamide der obigen Formel (IV) mit Schwefelkohlenstoff in Gegenwart eines Säureakzeptors, wie z. B. Kaliumhydroxid, und gegebenenfalls in Gegenwart eines Verdünnungsmittels, wie z. B. Dimethylformamid, bei Temperaturen zwischen 0 °C und 50 °C umsetzt und anschließend mit Phosgen oder Thionylchlorid, gegebenenfalls in Gegenwart eines Verdünnungsmittels, wie z. B. Methylenchlorid, bei Temperaturen zwischen 0 °C und 50 °C umsetzt (vgl. Arch. Pharm. 299 (1966), 174).

Die als Zwischenprodukte zu verwendenden 2-Halogenalkoxy-benzylsulfonamide sind durch die Formel (IV) definiert. In dieser Formel stehen $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Als Beispiele für die Zwischenprodukte der Formel (IV) seien genannt:
2-Difluormethoxy-, 2-Trifluormethoxy-, 2-Chlordifluormethoxy-, 2-Dichlorfluormethoxy-, 2-(2-Chlor-ethoxy-, 2-(2,2,2-Trifluorethoxy)-, 2-(1,1,2,2-Tetrafluorethoxy)-und 2-(2-Chlor-1,1,2-trifluorethoxy)-benzylsulfonamid, sowie die entsprechenden $\alpha$-Methyl-benzylsulfonamide.

Die Zwischenprodukte der Formel (IV) sind noch nicht in der Literatur beschrieben. Man erhält diese Verbindungen, wenn man 2-Halogenalkoxy-benzylsulfonsäurechloride der Formel (IX)

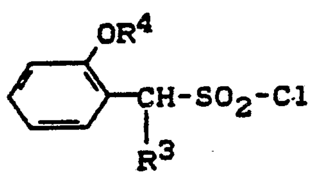

(IX)

in welcher
$R^3$ und $R^4$ die oben angegebenen Bedeutungen haben, mit Ammoniak in wenigstens der äquimolaren Menge, gegebenenfalls in Gegenwart eines Verdünnungsmittels, wie z. B. Wasser, Diethylether oder Tetrahydrofuran, bei Temperaturen zwischen -20 °C und +100 °C, vorzugsweise zwischen 0 °C und 80 °C umsetzt und nach üblichen Methoden aufarbeitet.

Die als Zwischenprodukte zu verwendenden 2-Halogenalkoxy-benzylsulfonsäurechloride sind durch die Formel (IX) definiert. In dieser Formel stehen $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Als Beispiele für die Zwischenprodukte der Formel (IX) seien genannt:
2-Difluormethoxy-, 2-Trifluormethoxy-, 2-Chlordifluormethoxy-, 2-Dichlorfluormethoxy-, 2-(2-Chlor-ethoxy)-, 2-(2,2,2-Trifluor-ethoxy)-, 2-(1,1,2,2-Tetrafluor-ethoxy)-und 2-(2-Chlor-1,1,2-trifluor-ethoxy)-benzylsulfonsäurechlorid sowie die entsprechenden $\alpha$-Methyl-benzylsulfonsäurechloride.

Die Zwischenprodukte der Formel (IX) sind noch nicht in der Literatur beschrieben. Man erhält diese Verbindungen, wenn man 2-Halogenalkoxy-benzylsulfonsäuren bzw. deren Salze der Formel (X)

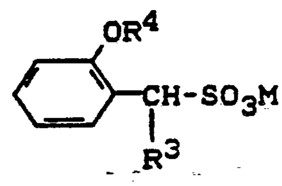

(X)

in welcher
M für Wasserstoff oder ein Alkalimetall steht und
$R^3$ und $R^4$ die oben angegebenen Bedeutungen haben, mit wenigstens der äquimolaren Menge eines Chlorierungsmittels, wie z. B. Phosphorpentachlorid, Phosphoroxychlorid oder Thionylchorid, gegebenenfalls in Gegenwart eines Verdünnungsmittels, wie z. B. Methylenchlorid, Chloroform oder Tetrachlormethan, bei Temperaturen zweischen 0 °C und 150 °C, vorzugsweise zwischen 20 °C und 100 °C umsetzt und nach üblichen Methoden aufarbeitet.

Die als Zwischenprodukte zu verwendenden 2-Halogenalkoxy-benzylsulfonsäuren bzw. deren Salze sind durch die Formel (X) definiert. In dieser Formel stehen $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden und M steht vorzugsweise wie auch insbesondere für Wasserstoff, Natrium oder Kalium.

Als Beispiele für die Zwischenprodukte der Formel (X) seien genannt:
2-Difluormethoxy-, 2-Trifluormethoxy-, 2-Chlordifluormethoxy, 2-Dichlorfluormethoxy, 2-(2-Chlor-ethoxy)-, 2-(2,2,2-Trifluor-ethoxy)-, 2-(1,1,2,2-Tetrafluor-ethoxy)-, und 2-(2-Chlor-1,1,2-trifluor-ethoxy)-benzylsulfonsäure sowie deren Natrium-und Kalium-Salze, ferner die entsprechenden α-Methyl-benzylsulfonsäuren sowie ihre Natrium-und Kalium-Salze.

Die Zwischenprodukte der Formel (X) sind noch nicht in der Literatur beschrieben. Man erhält diese Verbindungen, wenn man 2-Halogenalkoxy-benzylchloride der Formel (XI)

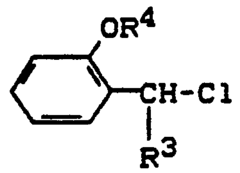

(XI)

in welcher
$R^3$ und $R^4$ die oben angegebenen Bedeutungen haben,
mit wenigstens der äquimolaren Menge Natrium-bzw. Kaliumthiosulfat in Wasser bei Temperaturen zwischen 20 °C und 110 °C umsetzt und nach üblichen Methoden aufarbeitet.

Die als Zwischenprodukte zu verwendenden 2-Halogenalkoxy-benzylchloride sind durch die Formel (XI) definiert. In dieser Formel stehen $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Als Beispiele für die Zwischenprodukte der Formel (XI) seien genannt:
2-Difluormethoxy-, 2-Trifluormethoxy-, 2-Chlordifluormethoxy-, 2-Dichlorfluormethoxy-, 2-(2-Chlor-ethoxy)-, 2-(1,1,2,2-Tetrafluor-ethoxy)-und 2-(2-Chlor-1,1,2-trifluor-ethoxy)-benzylchlorid sowie die entsprechenden α-Methyl-benzylchloride.

Die Zwischenprodukte der Formel (XI) sind zum Teil noch nicht in der Literatur beschrieben. Bisher unbekannt ist beispielsweise 2-Trifluormethoxy-benzylchorid. Man erhält diese Verbindungen, wenn man 2-Halogenalkoxy-toluole, z. B. 2-Trifluormethoxy-toluol, der Formel (XII)

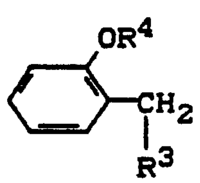

(XII)

in welcher
$R^3$ und $R^4$ die oben angegebenen Bedeutungen haben,
mit höchstens der äquimolaren Menge Chlor bei Temperaturen zwischen 50 °C und 180 °C unter UV-Bestrahlung umsetzt und nach üblichen Methoden aufarbeitet (vgl. z. B. DE-OS 21 50 955).

Die als Zwischenprodukte zu verwendenden 2-Halogenalkoxy-toluole sind durch die Formel (XII) definiert. In dieser Formel stehen $R^3$ und $R^4$ vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Als Beispiele für die Verbindungen der Formel (XII) seien genannt:
2-Difluormethoxy, 2-Trifluormethoxy-, 2-Chlordifluormethoxy-, 2-Dichlorfluormethoxy-, 2-(2-Chlor-ethoxy)-, 2-(1,1,2,2-Tetrafluor-ethoxy)-und 2-(2-Chlor-1,1,2-trifluor-ethoxy)-toluol sowie die entsprechenden α-Methyltoluole.

Die Zwischenprodukte der Formel (XII) sind bekannt und/oder können nach an sich bekannten Verfahren hergestellt werden (vgl. z. B. Tetrahedron Lett. 25 (1973), 2253 - 2256; DE-OS 21 50 955).

Die beim erfindungsgemäßen Herstellungsverfahren weiter als Ausgangsstoffe zu verwendenden Heteroarylamine sind durch die Formel (III) definiert. In dieser Formel stehen $R^1$, $R^2$, X, Y und Z vorzugsweise bzw. insbesondere für diejenigen Reste, die bereits im Zusammenhang mit der Beschreibung der erfindungsgemäßen Verbindungen der Formel (I) vorzugsweise bzw. als insbesondere bevorzugt angegeben wurden.

Beispiele für die Ausgangsstoffe der Formel (III) sind in der nachstehenden Tabelle 1 aufgeführt.

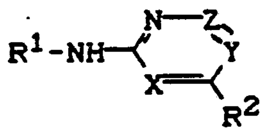 (III)

**Tabelle 1**

| $R^1$ | 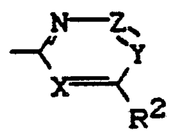 | $R^1$ | 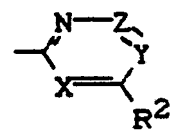 |
|---|---|---|---|
| $-CH_3$ | 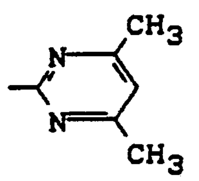 | $-CH_3$ | 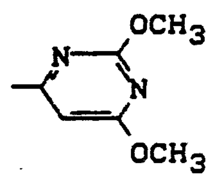 |
| $-CH_3$ | 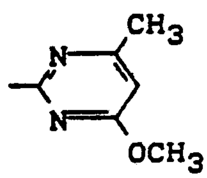 | $-CH_3$ | 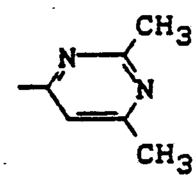 |
| $-CH_3$ | 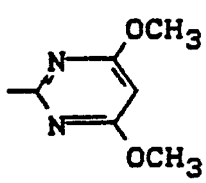 | $-CH_3$ | 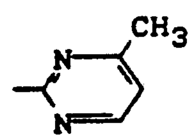 |

**Tabelle 1** - Fortsetzung

| $R^1$ | 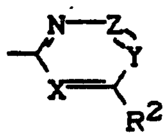 | $R^1$ | 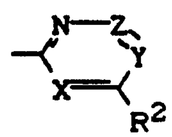 |
|---|---|---|---|
| $-CH_3$ | 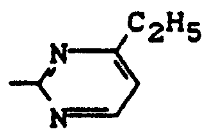 | $-CH_3$ | 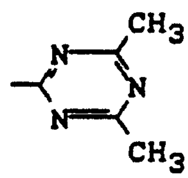 |
| $-CH_3$ | 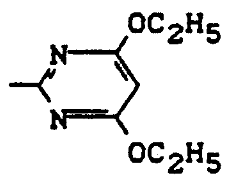 | $-CH_3$ | 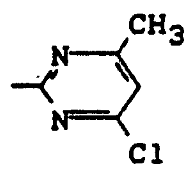 |
| $-CH_3$ | 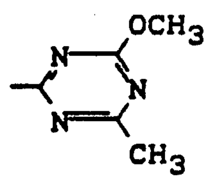 | $-CH_3$ | 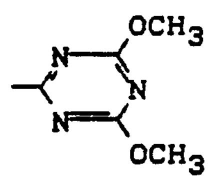 |
| $-CH_3$ | 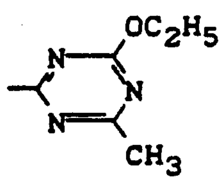 | $-CH_3$ | 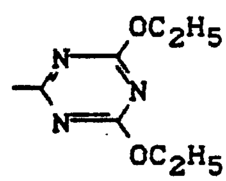 |
| $-CH_3$ | 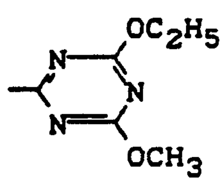 | $-CH_3$ | 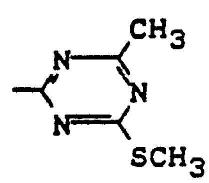 |
| $-CH_3$ | 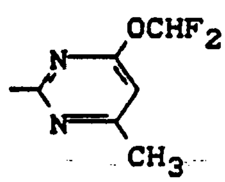 | $-CH_3$ | 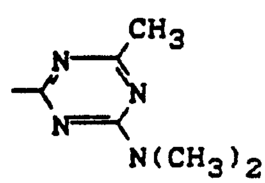 |

**Tabelle 1** - Fortsetzung

| $R^1$ | 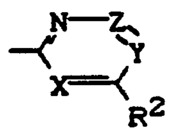 | $R^1$ | 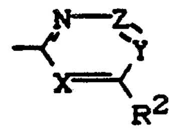 |
|---|---|---|---|
| $-CH_3$ | 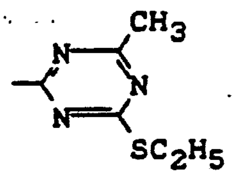 | $-CH_3$ | 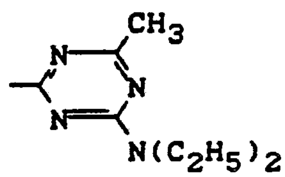 |
| $-CH_3$ | 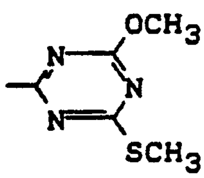 | $-CH_3$ | 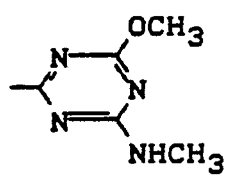 |
| $-CH_3$ | 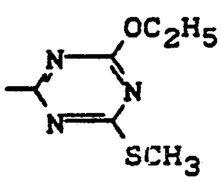 | $-CH_3$ | 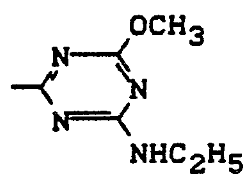 |
| $-CH_3$ | 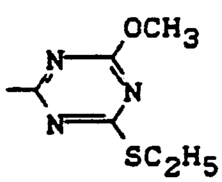 | $-CH_3$ | 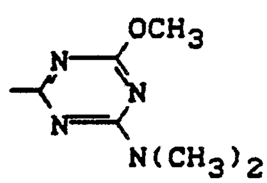 |
| $-CH_3$ | 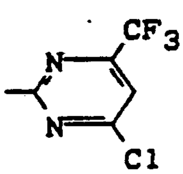 | $-CH_3$ | 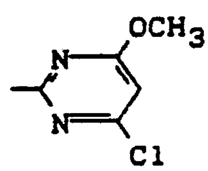 |
| $-CH_3$ | 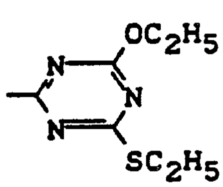 | $-CH_3$ | 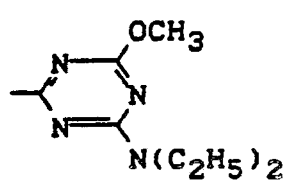 |

Tabelle 1 - Fortsetzung

| $R^1$ | 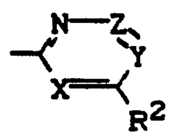 | $R^1$ | 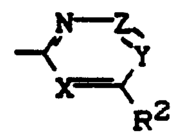 |
|---|---|---|---|
| $-CH_3$ | 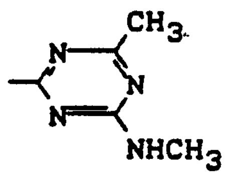 | $-CH_3$ | 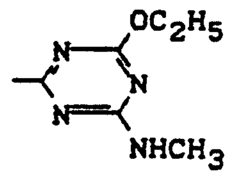 |
| $-CH_3$ | 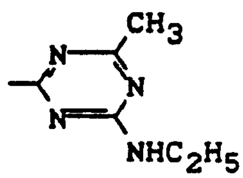 | $-CH_3$ | 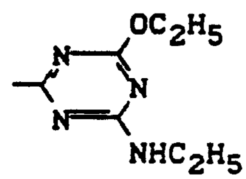 |
| $-CH_3$ | 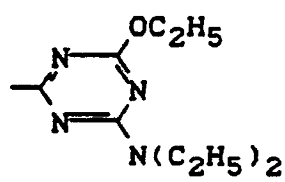 | $-CH_3$ | 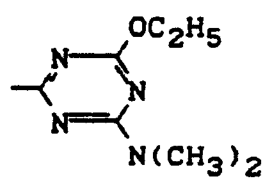 |
| $-CH_3$ | 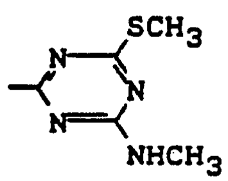 | $-CH_3$ | 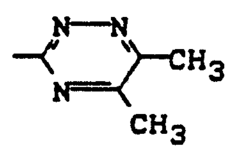 |
| $-CH_3$ | 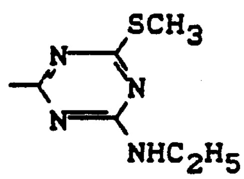 | $-CH_3$ | 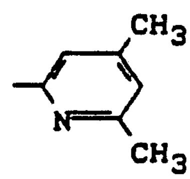 |
| $-CH_3$ | 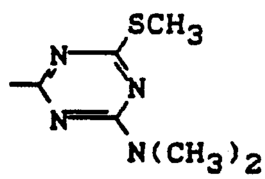 | $-CH_3$ | 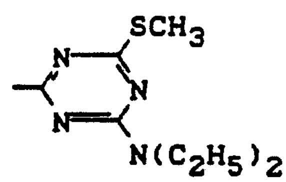 |
| $-CH_3$ | 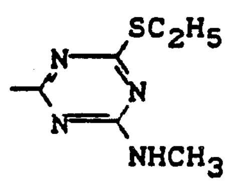 | $-CH_3$ | 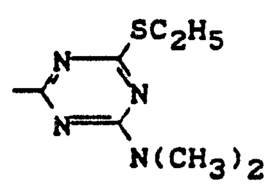 |

**Tabelle 1 - Fortsetzung**

| $R^1$ | 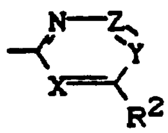 | $R^1$ | 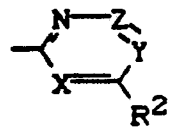 |
|---|---|---|---|
| $-CH_3$ | 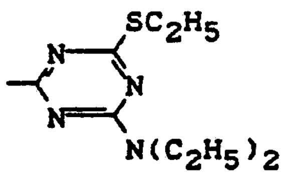 | $-CH_3$ | 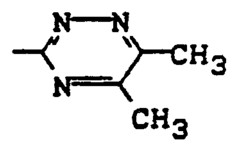 |
| H | 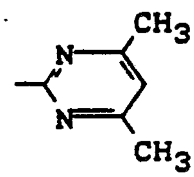 | H | 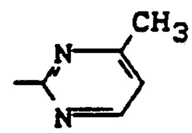 |
| H | 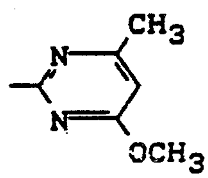 | H | 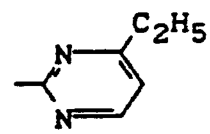 |
| H | 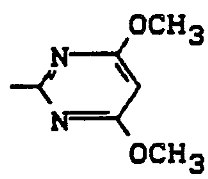 | H | 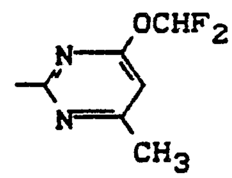 |
| H | 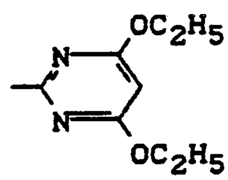 | $-CH_3$ | 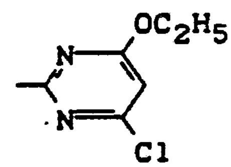 |
| H | 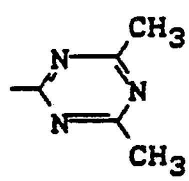 | H | 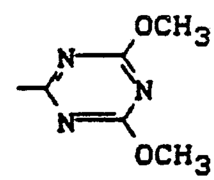 |
| H | 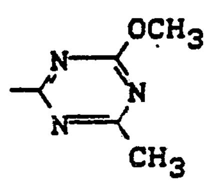 | H | 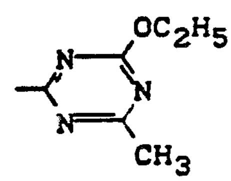 |

**Tabelle 1** - Fortsetzung

| $R^1$ | 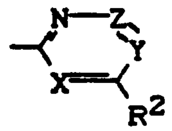 | $R^1$ | 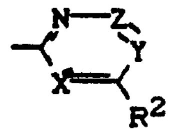 |
|---|---|---|---|
| H | 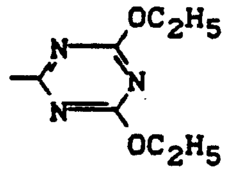 | $-CH_2-$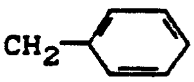 | 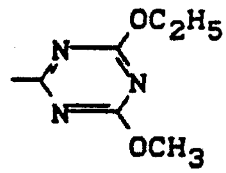 |
| H | 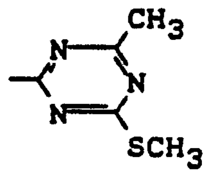 | | |
| $-C_2H_5$ | 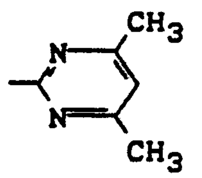 | $-C_2H_5$ | 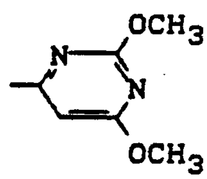 |
| $-C_2H_5$ | 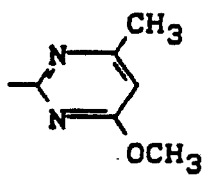 | $-C_2H_5$ | 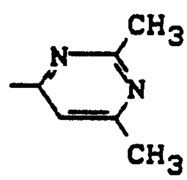 |
| $-C_2H_5$ | 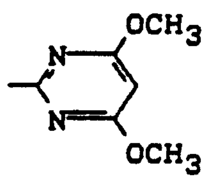 | $-C_2H_5$ | 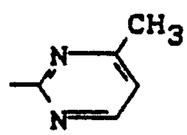 |
| $-C_2H_5$ | 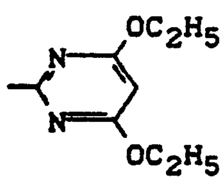 | $-CH_3$ | 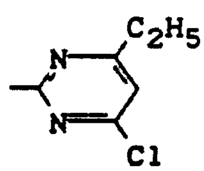 |
| $-CH_3$ | 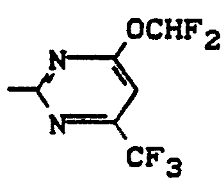 | $-CH_3$ | 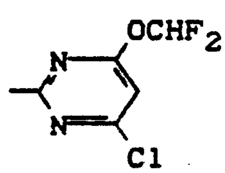 |

Tabelle 1 - Fortsetzung

| $R^1$ | 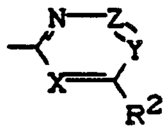 | $R^1$ | 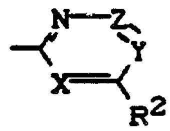 |
|---|---|---|---|
| $-C_2H_5$ | 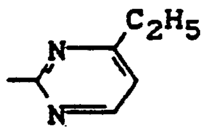 | $-C_2H_5$ | 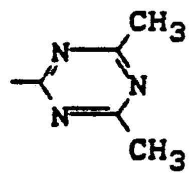 |
| $-C_2H_5$ | 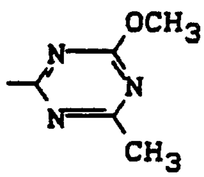 | $-C_2H_5$ | 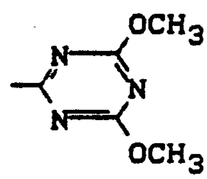 |
| $-C_2H_5$ | 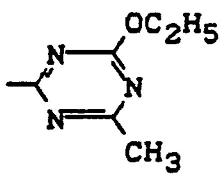 | $-C_2H_5$ | 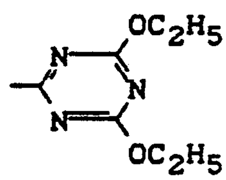 |
| $-C_2H_5$ | 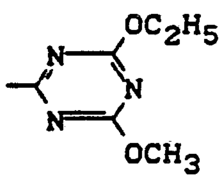 | $-C_2H_5$ | 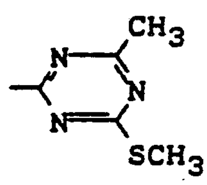 |
| $-C_2H_5$ | 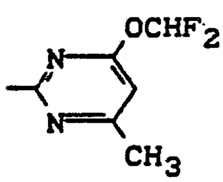 | $-C_2H_5$ | 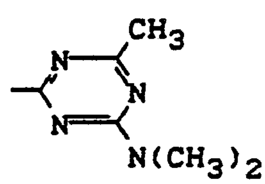 |
| $-C_2H_5$ | 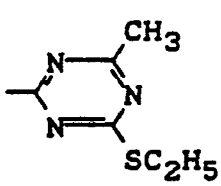 | $-C_2H_5$ | 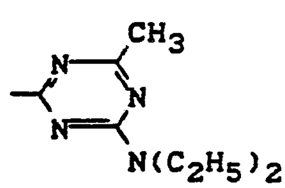 |
| $-C_2H_5$ | 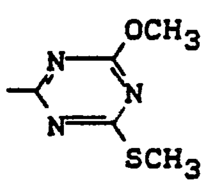 | $-C_2H_5$ | 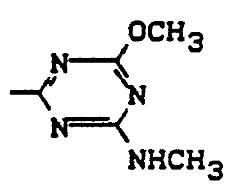 |

<u>Tabelle 1</u> - Fortsetzung

| $R^1$ | 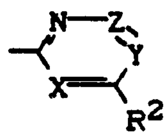 | $R^1$ | 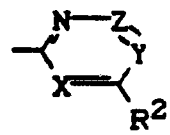 |
|---|---|---|---|
| $-C_2H_5$ | 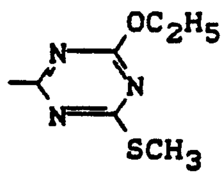 | $-C_2H_5$ | 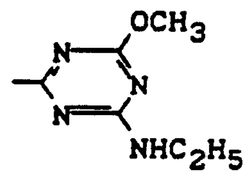 |
| $-C_2H_5$ | 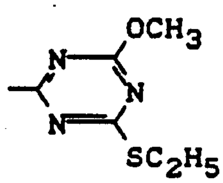 | $-C_2H_5$ | 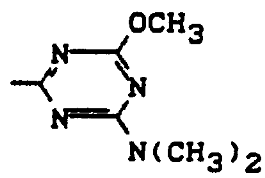 |
| $-C_2H_5$ | 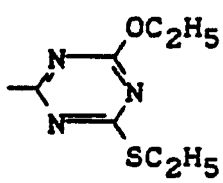 | $-C_2H_5$ | 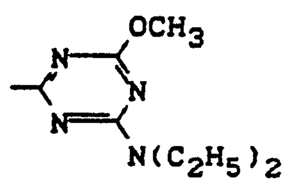 |
| $-C_2H_5$ | 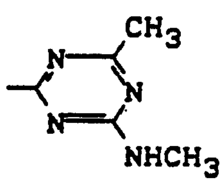 | $-C_2H_5$ | 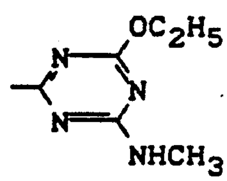 |
| $-C_2H_5$ | 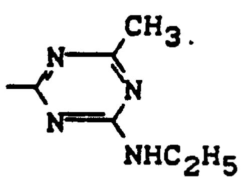 | $-C_2H_5$ | 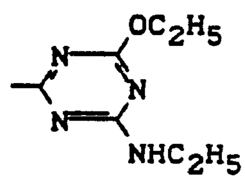 |
| $-C_2H_5$ | 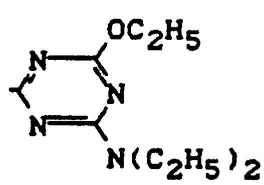 | $-C_2H_5$ | 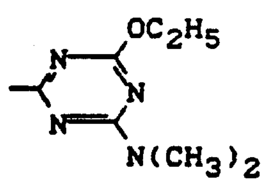 |
| $-C_2H_5$ | 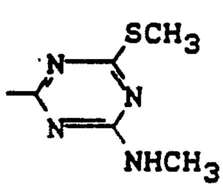 | $-C_2H_5$ | 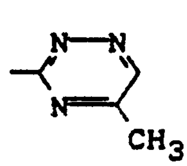 |

Tabelle 1 - Fortsetzung

| $R^1$ | 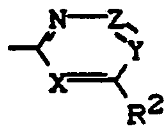 | $R^1$ | 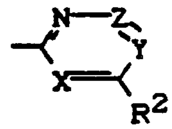 |
|---|---|---|---|
| $-C_2H_5$ | 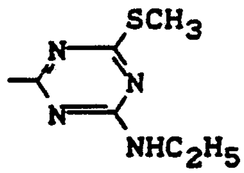 | $-C_2H_5$ | 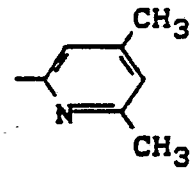 |
| $-C_2H_5$ | 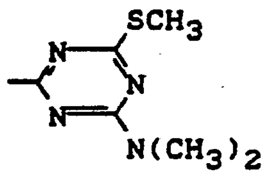 | $-C_2H_5$ | 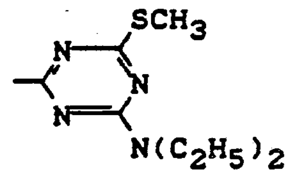 |
| $-C_2H_5$ | 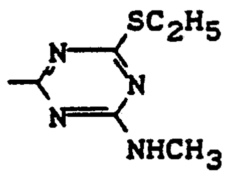 | $-C_2H_5$ | 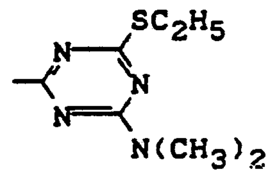 |
| $-C_2H_5$ | 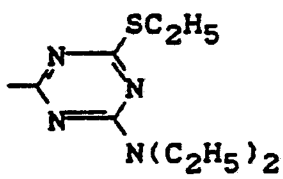 | $-C_2H_5$ | 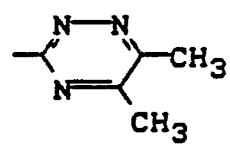 |
| $-C_2H_5$ | 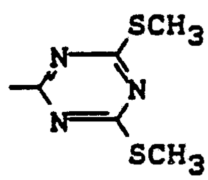 | $-C_2H_5$ | 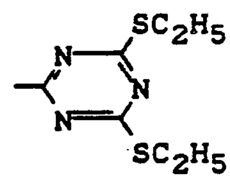 |
| $-CH_3$ | 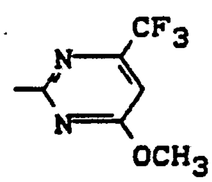 | $-CH_2$ 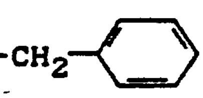 | 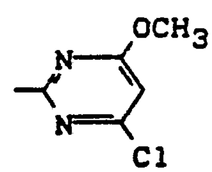 |
| $-CH_3$ | 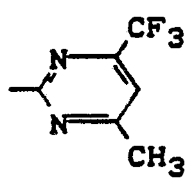 | $-CH_2$ 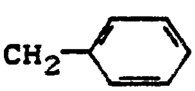 | 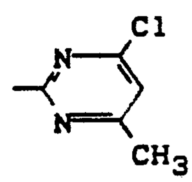 |

**Tabelle 1** - Fortsetzung

| $R^1$ | 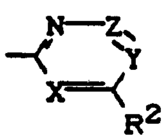 | $R^1$ | 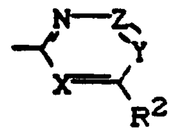 |
|---|---|---|---|
| $-CH_3$ | 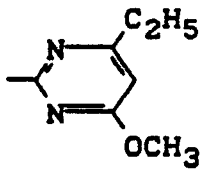 | $-CH_2-$ 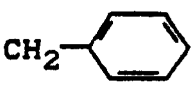 | 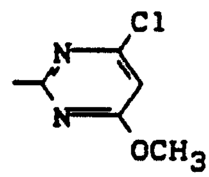 |
| $-C_3H_7-n$ | 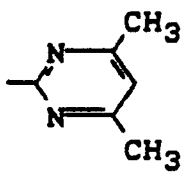 | $-C_3H_7-n$ | 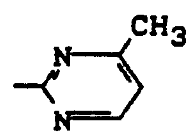 |
| $-C_3H_7-n$ | 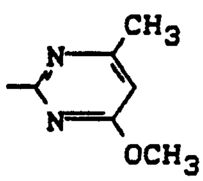 | $-C_3H_7-n$ | 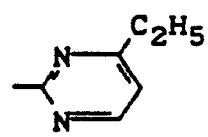 |
| $-C_3H_7-n$ | 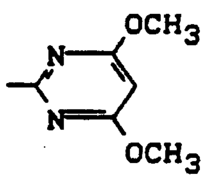 | $-C_3H_7-n$ | 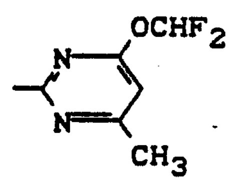 |
| $-C_3H_7-n$ | 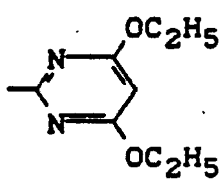 | $-C_3H_7-n$ | 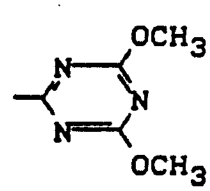 |
| $-C_3H_7-n$ | 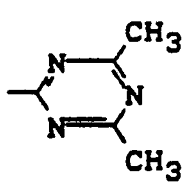 | $-C_3H_7-n$ | 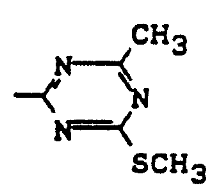 |
| $-C_3H_7-n$ | 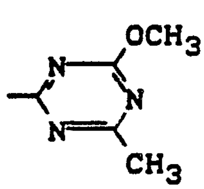 | $-C_3H_7-n$ | 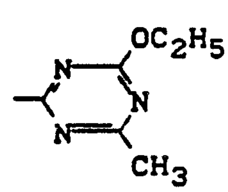 |

<u>Tabelle 1</u> - Fortsetzung

| $R^1$ | 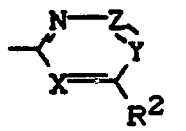 | $R^1$ | 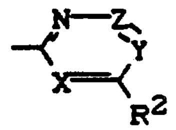 |
|---|---|---|---|
| $-C_3H_7$-n | 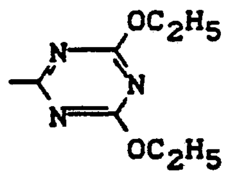 | $-C_3H_7$-n | 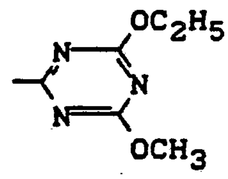 |
| $-CH_2-CH=CH_2$ | 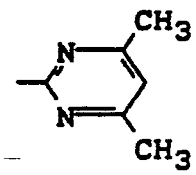 | $-CH_2-CH=CH_2$ | 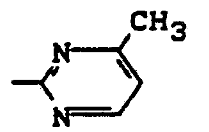 |
| $-CH_2-CH=CH_2$ | 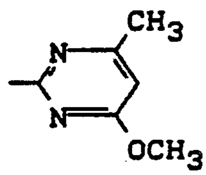 | $-CH_2-CH=CH_2$ | 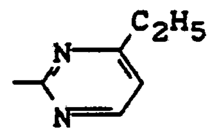 |
| $-CH_2-CH=CH_2$ | 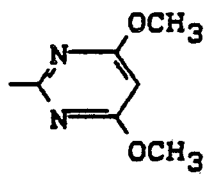 | $-CH_2-CH=CH_2$ | 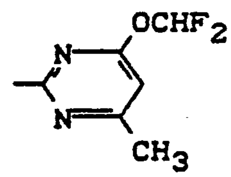 |
| $-CH_2-CH=CH_2$ | 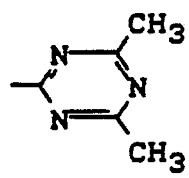 | $-CH_2-CH=CH_2$ | 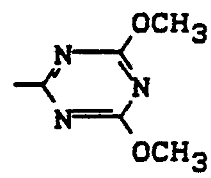 |
| $-CH_2-CH=CH_2$ | 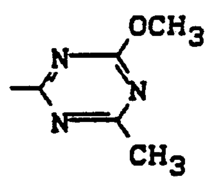 | $-CH_2-CH=CH_2$ | 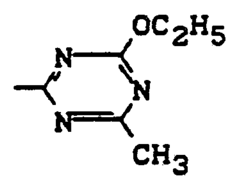 |
| $-CH_2-CH=CH_2$ | 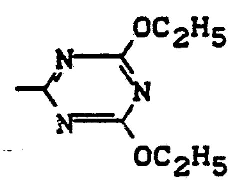 | $-CH_2-CH=CH_2$ | 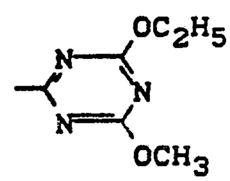 |

Tabelle 1 - Fortsetzung

| $R^1$ | 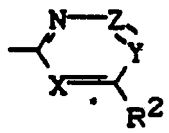 | $R^1$ | 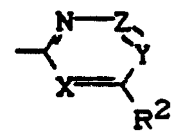 |
|---|---|---|---|
| $-CH_2-CH=CH_2$ | 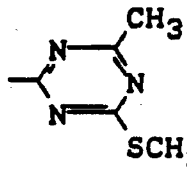 | H | 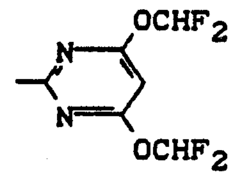 |
| H | 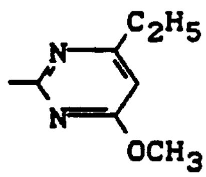 | H | 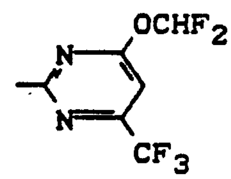 |
| H | 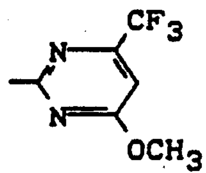 | H | 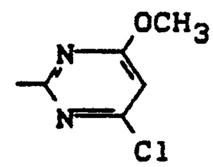 |
| H | 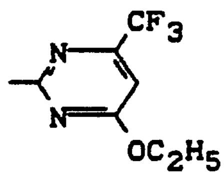 | H | 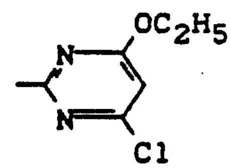 |
| H | 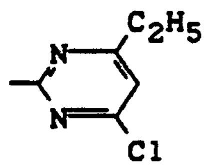 | H | 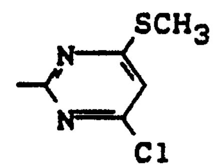 |
| H | 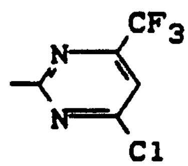 | H | 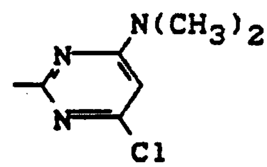 |
| H | 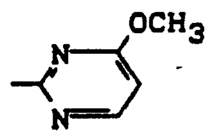 | | |

Die Verbindungen der Formel (III) sind bekannt und können nach an sich bekannten Verfahren hergestellt werden (vergl. z. B. Chem. Pharm. Bull. 11 (1963), 1382 und US-PS 4 299 960, EP-A 121 082, EP-A 125 205, EP-A 126 711 und EP-A 152 378).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoff wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und

Diglykoldimethylether, Tetrahydrofuran und Dioxan, Ketone wie Aceton, Methyl-ether. Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird gegebenenfalls in Gegenwart eines Katalysators durchgeführt. Hierzu gehören insbesondere aliphatische, aromatische oder heterocyclische Amine, wie Triethylamin, N,N-Dimethylanilin, Pyridin, 2-Methyl-5-ethyl-pyridin, 4-Dimethylamino-pyridin, Diazabicyclooctan (DABCO), Diazabicyclo nonen (DBN) und Diazabicycloundecen-(DBU).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren wir im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man je Mol Heteroarylamin der Formel (III) im allgemeinen 1,0 bis 3,0 Mol, vorzugsweise 1,0 bis 2,0 Mol 2-Halogenalkoxy-benzolsulfonyl-iso(thio)-cyanat der Formel (II) ein.

Die Ausgangsstoffe der Formeln (II) und (III) und gegebenenfalls der Katalysator und das Verdünnungsmittel werden im allgemeinen bei Raumtemperatur oder unter leichter Aussenkühlung zusammengegeben und das Reaktionsgemisch wird gegebenenfalls bei erhöhter Temperatur bis zum Reaktionsende gerührt.

Die Aufarbeitung und Isolierung der neuen Verbindungen der Formel (I) erfolgt nach üblichen Methoden: Soweit die Verbindungen der Formel (I) kristallin anfallen, werden sie durch Absaugen isoliert. Andernfalls wird - gegebenenfalls nach Einengen - Wasser und ein mit Wasser praktisch nicht mischbares organisches Lösungs mittel dazugegeben, nach Durchschütteln die organische Phase abgetrennt, getrocknet, filtriert und eingeengt, wobei die Produkte der Formel (I) im Rückstand verbleiben.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Opomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie-und Gleisanlagen und auf Wegen und Plätzen mit und ohne Baumbewuchs. Ebenso können die Verbindungen zur Unkrautbekämpfung in Dauerkulturen, z. B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die neuen Wirkstoffe der Formel (I) eignen sich zur selektiven Bekämpfung dikotyler Unkräuter in monokotylen Kulturen im Vorauflauf-und im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Naturstoffe und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Also flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 1-(4-Methyl-phenyl)-3-(1-methyl-1-phenyl-ethyl)-harnstoff, 2-Chlor-N-{[4-methoxy-6-methyl-1,3,5-triazin-2-yl)-amino]carbonyl}-benzolsulfonamid, Ethyl-2-{[(4-Chlor-6-methoxy-2-pyrimidyl)aminocarbonyl]-aminosulfonyl}-benzoat, 2-Ethylamino-6 (1,1-dimethylethyl-amino)-4-methylthio-1,3,5-triazin, 2-Chlor-4-ethylamino-6-(1-methyl-ethyl)-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 5-(2-Chlor-4-trifluormethyl-phenoxy)-N-methylsulfonyl-2-nitrobenzamid, (2-Ethoxy-1-methyl-2-oxoethyl)-5-(2-chlor-4-trifluormethyl-phenoxy)-2-nitrobenzoat, 2-[4-(2,4-Dichlorphenoxy)-phenoxy]-propionsäuremethylester, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-pro pionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, 2-[5-Methyl-5-(1-methylethyl)-4-oxo-2-imidazolin-2-yl]-3-pyridincarbonsäure, 2-(1-Ethoxyamino-butyliden)-5-(2-ethylthiopropyl)-1,3-cyclohexandion, [(4-Amino-3,5-dichlor-6-fluor-2-pyridyl)-oxy]-essigsäure, 3-Isopropyl-2,1,3-benzothiadiazinon-(4)-2,2-dioxid, 2-(2-Benzothiazolyloxy)-N-methyl-N-phenyl-acetamid, S-Ethyl-N,N-dipropylthiocarbamat, 1-Ethylthiocarbonyl-hexahydro-1H-azepin, Propionsäure-3,4-dichloranilid, Chloressigsäure-N-(2,6-diethyl-phenyl)-N-methoxymethyl-amid, Chloressigsäure-N-(2-ethyl-6-methyl-phenyl)-N-(2-methoxy-1-methyl-ethyl)-amid, Chloressigsäure-N-(2,6-dimethyl-phenyl)-N-(1H-pyrazol-1-yl-methyl)-amid, Chloressigsäure-N-(2,6-diethylphenyl)-N-(2-propoxyethyl)-amid, 3,7-Dichlor-8-chinolincarbonsäure, 4-(2,4-Dichlor-phenoxy)-2-methoxy-anilin, 2-(-((6-Chlor-benzoxazol-2-yl)-oxy)-phenoxy)-propionsäure-ethylester, (2,4-Dichlorphenyl)-(1,3-dimethyl-5-((4-methyl-phenyl)-sulfonyl-oxy)-1H-pyrazol-4-yl)-methanon und O-(6-Chlor-3-phenyl-4-pyridazinyl)-S-octylthiocarbonat. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten unde Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 5 kg Wirkstoff pro Hektar Bodenfläche, vorzugsweise zwischen 0,01 und 1 kg pro ha.

Die Herstellung und die Verwendung der erfindungsgemäßen Wirkstoffe geht aus den nachfolgenden Beispielen hervor.

## Herstellungsbeispiele

### Beispiel 1

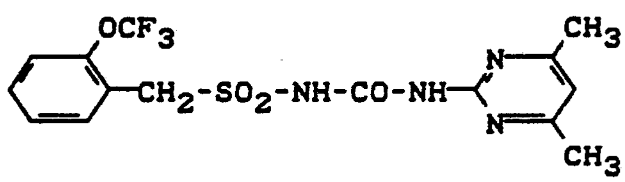

Eine Lösung von 2-Trifluormethoxy-benzylsulfonyl-isocyanat in 100 ml Methylenchlorid (Herstellung vgl. Beispiel (II-I); die gemäß Beispiel (II-I) erhaltene Substanzmenge wird hier eingesetzt) wird mit 2,5 g (0,02 Mol) 2-Amino-4,6-dimethyl-pyrimidin 12 Stunden bei 20 °C gerührt. Dann wird eingeengt, der Rückstand mit Diethylether digeriert und das kristallin angefallene Reaktionsprodukt durch Absaugen isoliert.

Man erhält 6,0 g (74 % der Theorie) 1-(2-Trifluormethoxy-benzylsulfonyl)-3-(4,6-dimethyl-pyrimidin-2-yl)harnstoff vom Schmelzpunkt 151 °C (Zersetzung).

Nach dem in Beispiel 1 exemplarisch beschriebenen Verfahren können die in der nachstehenden Tabelle 2 aufgeführten Verbindungen der Formel (I) hergestellt werden:

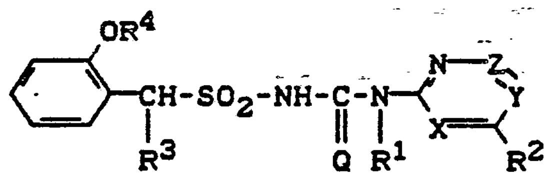 (I)

<u>Tabelle 2</u>

| Beisp.-Nr. | $R^1$ | $R^3$ | $R^4$ | Q | 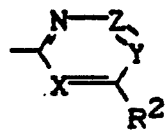 | Schmelzpunkt (°C) |
|---|---|---|---|---|---|---|
| 2 | H | H | $CF_3$ | O | 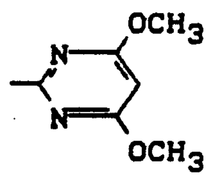 | 170 (Zers.) |
| 3 | H | H | $CF_3$ | O | 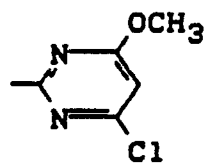 | 178 (Zers.) |
| 4 | H | H | $CF_3$ | O | 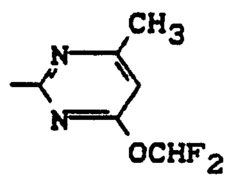 | 154 (Zers.) |
| 5 | H | H | $CF_3$ | O | 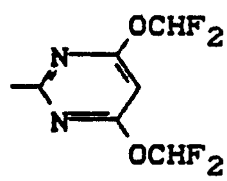 | 158 |
| 6 | H | H | $CF_3$ | O | 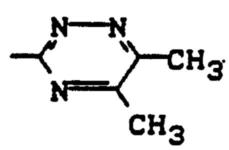 | 141 (Zers.) |
| 7 | $CH_3$ | H | $CF_3$ | O | 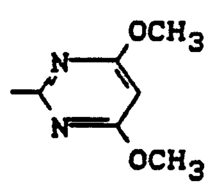 | 98 (Zers.) |
| 8 | H | H | $CF_3$ | O | 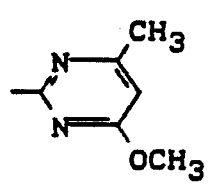 | 164 |

<u>Tabelle 2</u> - Fortsetzung

| Beisp.-Nr. | $R^1$ | $R^3$ | $R^4$ | Q | 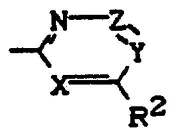 | Schmelz-punkt(° C) |
|---|---|---|---|---|---|---|
| 9 | $CH_3$ | H | $CF_3$ | O | 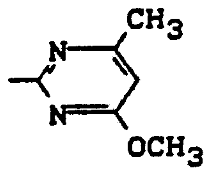 | |
| 10 | $CH_3$ | H | $CF_3$ | O | 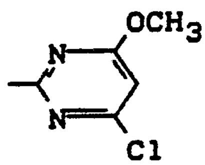 | |
| 11 | H | H | $CF_3$ | O | 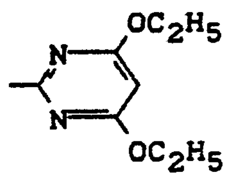 | 156 |
| 12 | H | H | $CF_3$ | O | 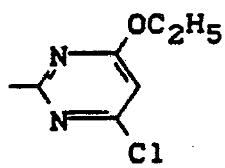 | 170 |
| 13 | H | H | $CF_3$ | O | 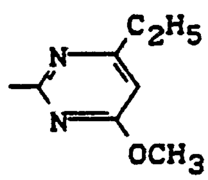 | |
| 14 | H | H | $CF_3$ | O | 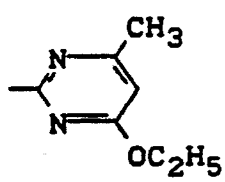 | |
| 15 | $CH_3$ | H | $CF_3$ | O | 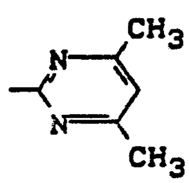 | |

Tabelle 2 - Fortsetzung

| Beisp.-Nr. | $R^1$ | $R^3$ | $R^4$ | Q | 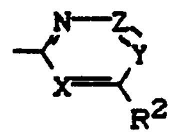 | Schmelz-punkt (°C) |
|---|---|---|---|---|---|---|
| 16 | H | H | $CF_3$ | O | 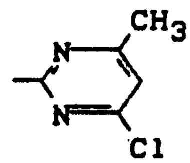 | |
| 17 | H | H | $CF_3$ | O | 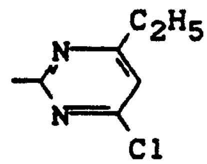 | |
| 18 | H | H | $CF_3$ | O | 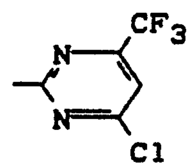 | |
| 19 | H | H | $CF_3$ | O | 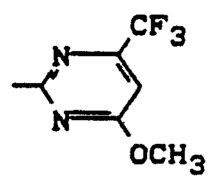 | |
| 20 | H | H | $CHF_2$ | O | 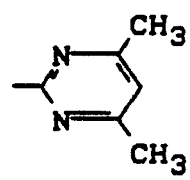 | |
| 21 | H | H | $CHF_2$ | O | 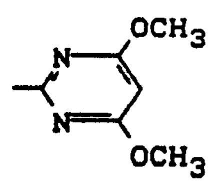 | 135 |
| 22 | $CH_3$ | H | $CHF_2$ | O | 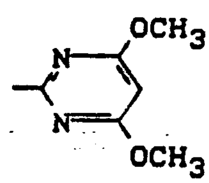 | |

**Tabelle 2 - Fortsetzung**

| Beisp.-Nr. | $R^1$ | $R^3$ | $R^4$ | Q | 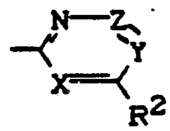 | Schmelzpunkt(°C) |
|---|---|---|---|---|---|---|
| 23 | H | H | $CHF_2$ | O | 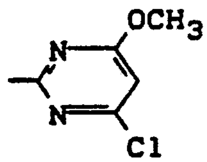 | |
| 24 | H | H | $CHF_2$ | O | 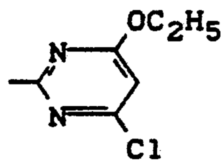 | |
| 25 | H | $CH_3$ | $CF_3$ | O | 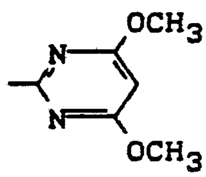 | |
| 26 | H | H | $CHF_2$ | O | 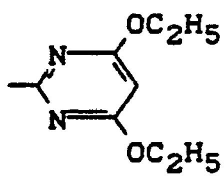 | |
| 27 | H | H | $CF_3$ | O | 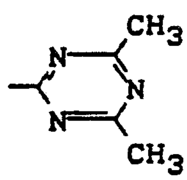 | 170 |
| 28 | H | H | $CF_3$ | O | 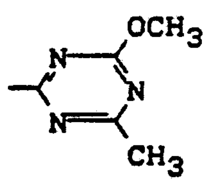 | 163 |
| 29 | $CH_3$ | H | $CF_3$ | O | 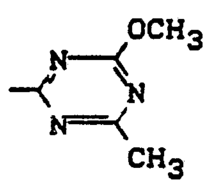 | 157 |

Tabelle 2 - Fortsetzung

| Beisp.-Nr. | $R^1$ | $R^3$ | $R^4$ | Q | 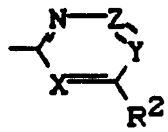 | Schmelzpunkt(°C) |
|---|---|---|---|---|---|---|
| 30 | H | $CH_3$ | $CF_3$ | O | 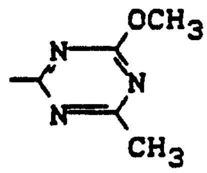 | |
| 31 | H | H | $CF_3$ | O | 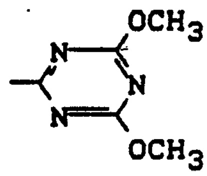 | 147 |
| 32 | $CH_3$ | H | $CF_3$ | O | 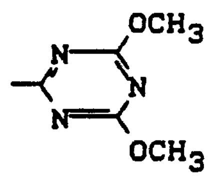 | |
| 33 | H | H | $CHF_2$ | O | 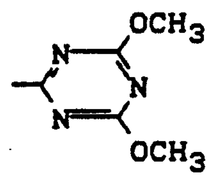 | 142 |
| 34 | H | H | $CClF_2$ | O | 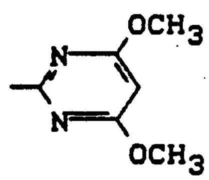 | |
| 35 | H | H | $CFCl_2$ | O | 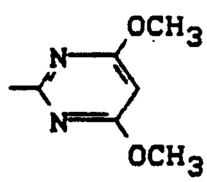 | |
| 36 | H | H | $CH_2CH_2Cl$ | O | 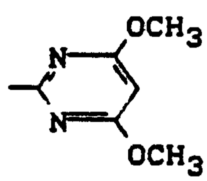 | |

Tabelle 2 - Fortsetzung

| Beisp.-Nr. | $R^1$ | $R^3$ | $R^4$ | Q | 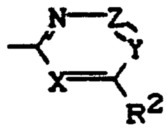 | Schmelzpunkt(°C) |
|---|---|---|---|---|---|---|
| 37 | H | H | $CH_2CH_2Cl$ | O | 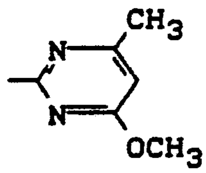 | |
| 38 | H | H | $CH_2CH_2Cl$ | O | 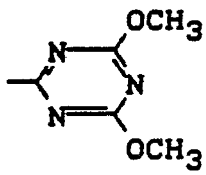 | |
| 39 | H | H | $CF_2CHF_2$ | O | 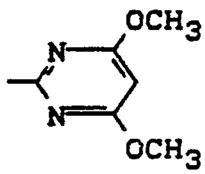 | |
| 40 | H | H | $CF_2CHF_2$ | O | 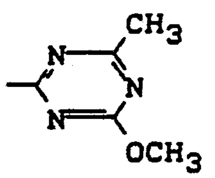 | |
| 41 | H | H | $CF_2CHFCl$ | O | 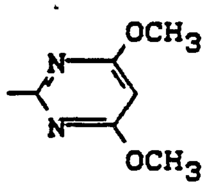 | |
| 42 | H | H | $CF_2CHFCl$ | O | 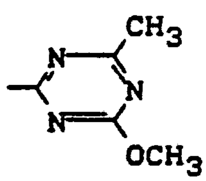 | |
| 43 | H | H | $CF_3$ | O | 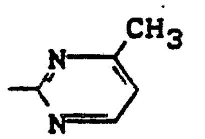 | 193 |

Tabelle 2 - Fortsetzung

| Beisp.-Nr. | $R^1$ | $R^3$ | $R^4$ | Q | 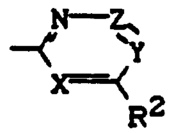 | Schmelz-punkt(°C) |
|---|---|---|---|---|---|---|
| 44 | H | H | $CF_3$ | O | 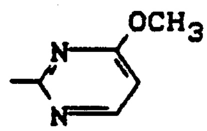 | 106 |
| 45 | H | H | $CF_3$ | O | 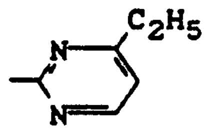 | |
| 46 | H | H | $CF_3$ | O | 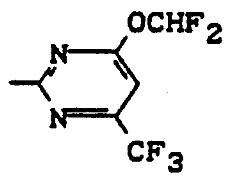 | 132 |
| 47 | H | H | $CF_3$ | O | 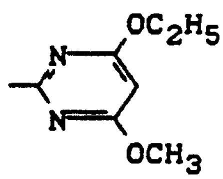 | |
| 48 | H | H | $CF_3$ | O | 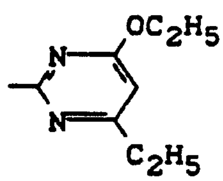 | |
| 49 | H | H | $CF_3$ | O | 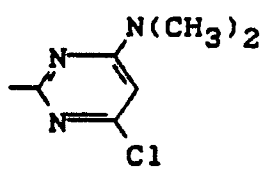 | |
| 50 | H | H | $CHF_2$ | O | 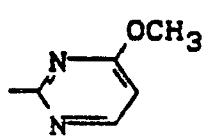 | |

<u>Tabelle 2</u> - Fortsetzung

| Beisp.-Nr. | $R^1$ | $R^3$ | $R^4$ | Q | 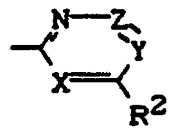 | Schmelzpunkt (°C) |
|---|---|---|---|---|---|---|
| 51 | H | H | $CHF_2$ | O | 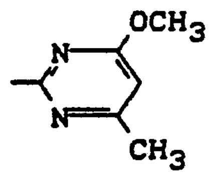 | |
| 52 | H | H | $CHF_2$ | O | 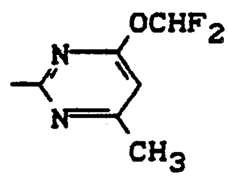 | |
| 53 | H | H | $CHF_2$ | O | 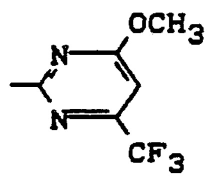 | |
| 54 | H | H | $CHF_2$ | O | 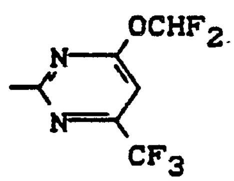 | |
| 55 | H | H | $CHF_2$ | O | 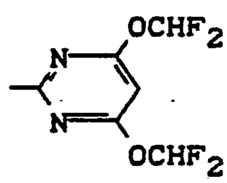 | |
| 56 | H | H | $CHF_2$ | O | 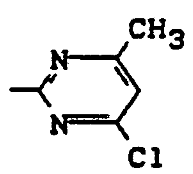 | |
| 57 | H | H | $CHF_2$ | O | 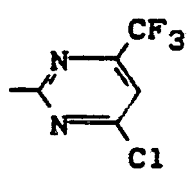 | |

<u>Tabelle 2</u> - Fortsetzung

| Beisp.-Nr. | $R^1$ | $R^3$ | $R^4$ | Q | 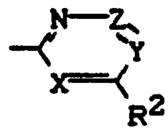 | Schmelzpunkt (° C) |
|---|---|---|---|---|---|---|
| 58 | H | H | $CHF_2$ | O | 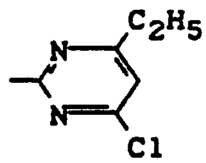 | |
| 59 | H | H | $CHF_2$ | O | 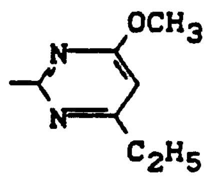 | |
| 60 | H | H | $CHF_2$ | O | 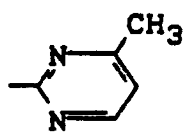 | |
| 61 | H | H | $CHF_2$ | O | 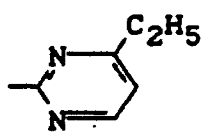 | |
| 62 | H | H | $CHF_2$ | O | 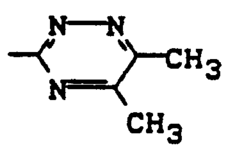 | |
| 63 | H | H | $CHF_2$ | O | 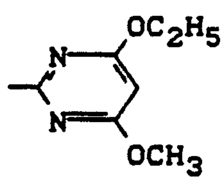 | |
| 64 | H | H | $CHF_2$ | O | 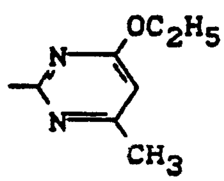 | |

Tabelle 2 - Fortsetzung

| Beisp.-Nr. | $R^1$ | $R^3$ | $R^4$ | Q | 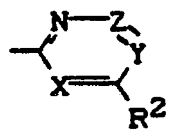 | Schmelzpunkt (°C) |
|---|---|---|---|---|---|---|
| 65 | H | H | $CHF_2$ | O | 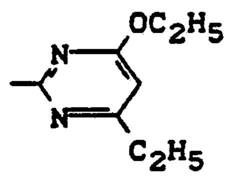 | |
| 66 | H | H | $CHF_2$ | O | 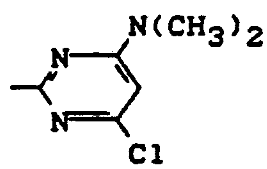 | |
| 67 | H | H | $CHF_2$ | O | 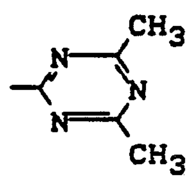 | |
| 68 | $CH_3$ | H | $CHF_2$ | O | 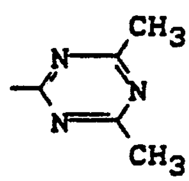 | |
| 69 | $CH_3$ | H | $CF_3$ | O | 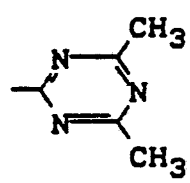 | |
| 70 | $CH_3$ | H | $CHF_2$ | O | 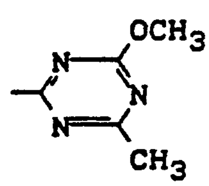 | 132 |
| 71 | H | H | $CHF_2$ | O | 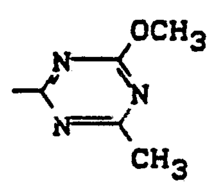 | 108 |

Tabelle 2 - Fortsetzung

| Beisp.-Nr. | $R^1$ | $R^3$ | $R^4$ | Q | 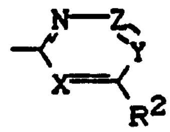 | Schmelzpunkt (°C) |
|---|---|---|---|---|---|---|
| 72 | $CH_3$ | H | $CHF_2$ | O | 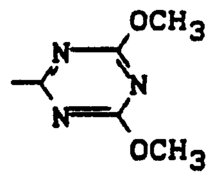 | |
| 73 | $CH_3$ | H | $CF_3$ | O | 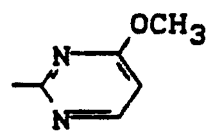 | |
| 74 | $CH_3$ | H | $CHF_2$ | O | 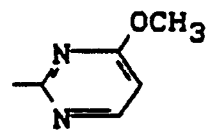 | |
| 75 | H | H | $CH_2CH_2Cl$ | O | 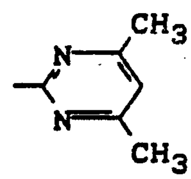 | |
| 76 | H | H | $CH_2CH_2Cl$ | O | 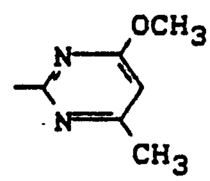 | |
| 77 | H | H | $CH_2CH_2Cl$ | O | 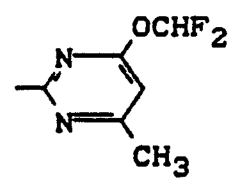 | |
| 78 | H | H | $CH_2CH_2Cl$ | O | 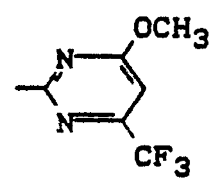 | |

Tabelle 2 - Fortsetzung

| Beisp.-Nr. | $R^1$ | $R^3$ | $R^4$ | Q | 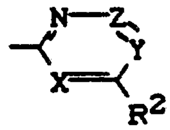 | Schmelzpunkt (°C) |
|---|---|---|---|---|---|---|
| 79 | H | H | $CH_2CH_2Cl$ | O | 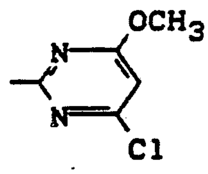 | |
| 80 | H | H | $CH_2CH_2Cl$ | O | 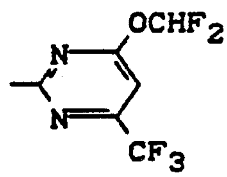 | |
| 81 | H | H | $CH_2CH_2Cl$ | O | 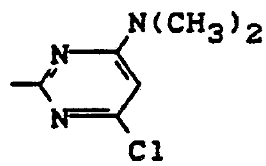 | |
| 82 | H | H | $CH_2CH_2Cl$ | O | 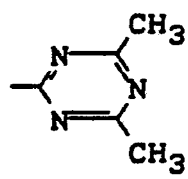 | |
| 83 | H | H | $CH_2CH_2Cl$ | O | 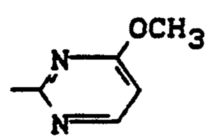 | |
| 84 | $CH_3$ | H | $CH_2CH_2Cl$ | O | 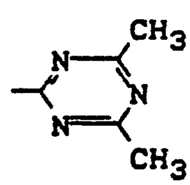 | |
| 85 | $CH_3$ | H | $CH_2CH_2Cl$ | O | 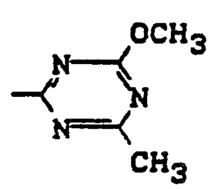 | |

**Tabelle 2** - Fortsetzung

| Beisp.-Nr. | $R^1$ | $R^3$ | $R^4$ | Q | 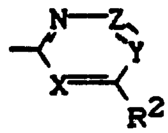 | Schmelzpunkt (°C) |
|---|---|---|---|---|---|---|
| 86 | $CH_3$ | H | $CH_2CH_2Cl$ | O | 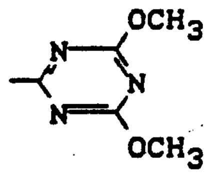 | |
| 87 | H | H | $CH_2$-$CF_3$ | O | 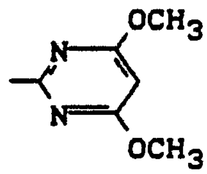 | |
| 88 | $CH_3$ | H | $CH_2$-$CF_3$ | O | 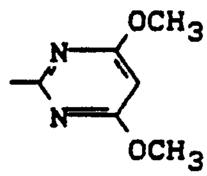 | |
| 89 | H | H | $CH_2$-$CF_3$ | O | 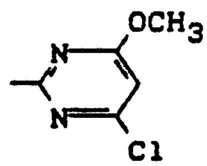 | |
| 90 | H | H | $CH_2$-$CF_3$ | O | 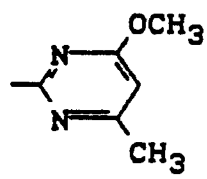 | |
| 91 | H | H | $CH_2$-$CF_3$ | O | 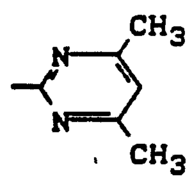 | |
| 92 | H | H | $CH_2$-$CF_3$ | S | 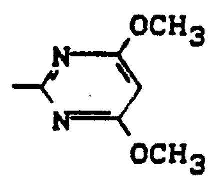 | |

<u>Tabelle 2</u> - Fortsetzung

| Beisp.-Nr. | $R^1$ | $R^3$ | $R^4$ | Q | 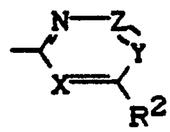 | Schmelzpunkt (°C) |
|---|---|---|---|---|---|---|
| 93 | H | H | $CH_2CH_2Cl$ | S | 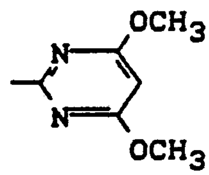 | |
| 94 | $CH_3$ | H | $CH_2CH_2Cl$ | S | 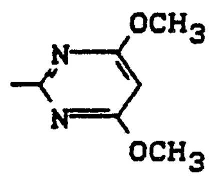 | |
| 95 | H | H | $CHF_2$ | S | 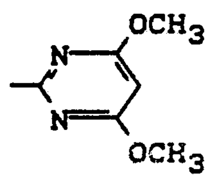 | |
| 96 | $CH_3$ | H | $CHF_2$ | S | 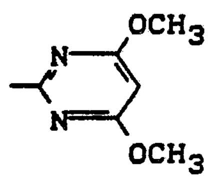 | |
| 97 | H | H | $CF_3$ | S | 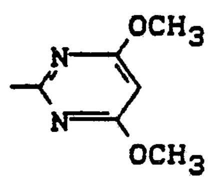 | |
| 98 | $CH_3$ | H | $CF_3$ | S | 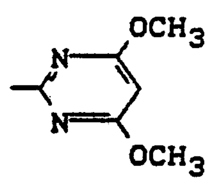 | |
| 99 | H | H | $CF_3$ | S | 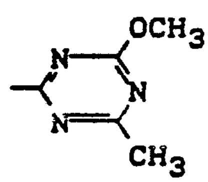 | |

Tabelle 2 - Fortsetzung

| Beisp.-Nr. | $R^1$ | $R^3$ | $R^4$ | Q | 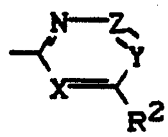 | Schmelzpunkt (°C) |
|---|---|---|---|---|---|---|
| 100 | $CH_3$ | H | $CF_3$ | S | 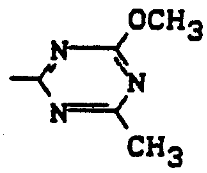 | |
| 101 | H | H | $CHF_2$ | S | 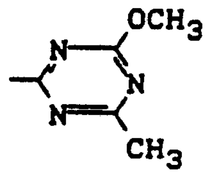 | |
| 102 | $CH_3$ | H | $CHF_2$ | S | 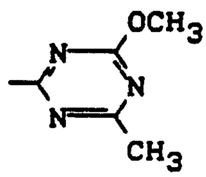 | |
| 103 | H | H | $CH_2CH_2Cl$ | S | 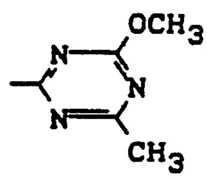 | |
| 104 | $CH_3$ | H | $CH_2CH_2Cl$ | S | 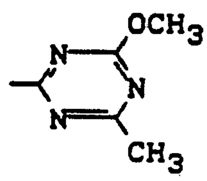 | |
| 105 | H | H | $CF_3$ | O | 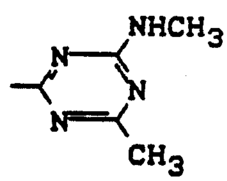 | |
| 106 | H | H | $CF_3$ | O | 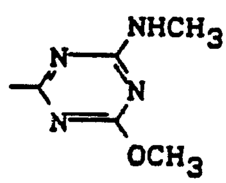 | |

**Tabelle 2** - Fortsetzung

| Beisp.-Nr. | $R^1$ | $R^3$ | $R^4$ | Q | 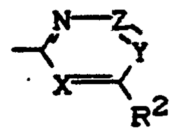 $R^2$ | Schmelzpunkt (° C) |
|---|---|---|---|---|---|---|
| 107 | $CH_3$ | H | $CF_3$ | O | 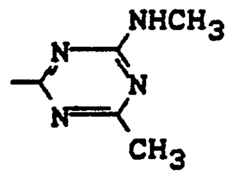 | |
| 108 | $CH_3$ | H | $CF_3$ | O | 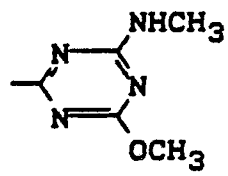 | |
| 109 | H | H | $CF_3$ | O | 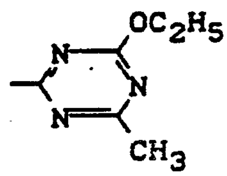 | |
| 110 | H | H | $CHF_2$ | O | 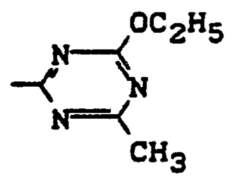 | |
| 111 | $CH_3$ | H | $CF_3$ | O | 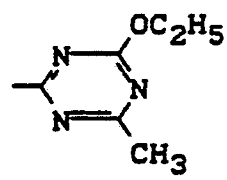 | |
| 112 | $CH_3$ | H | $CHF_2$ | O | 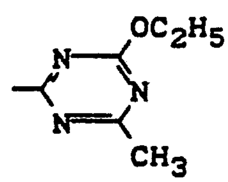 | |
| 113 | H | H | $CF_3$ | O | 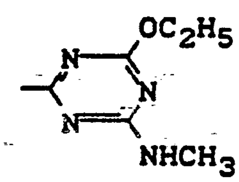 | |

Tabelle 2 - Fortsetzung

| Beisp.-Nr. | $R^1$ | $R^3$ | $R^4$ | Q | 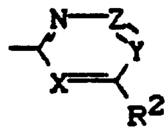 $R^2$ | Schmelzpunkt (°C) |
|---|---|---|---|---|---|---|
| 114 | $CH_3$ | H | $CF_3$ | O | 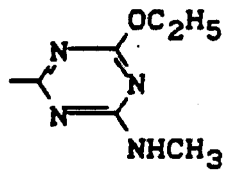 | |
| 115 | H | H | $CHF_2$ | O | 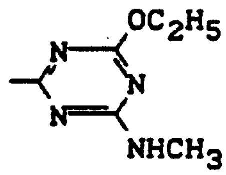 | |
| 116 | $CH_3$ | H | $CHF_2$ | O | 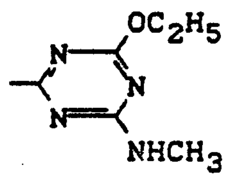 | |
| 117 | H | H | $CF_3$ | O | 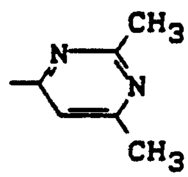 | 143 |

Ausgangsstoffe der Formel (II)

Beispiel (II-I)

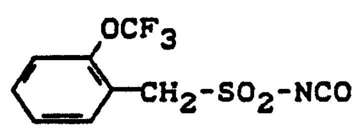

Eine Mischung aus 8,9 g (0,035 Mol) 2-Trifluormethoxybenzylsulfonsäureamid, 3,5 g (0,035 Mol) n-Butylisocyanat, 0,2 g Diaza-bicyclo-[2,2,2]-octan (DABCO) und 150 ml wasserfreiem Xylol wird auf Rückflußtemperatur erhitzt und für zwei Stunden wird ein schwacher Phosgen-Strom durchgeleitet. Es wird noch 30 Minuten bei Rückfluß nachgerührt, dann abgekühlt, filtriert und eingeengt. Der Rückstand wird in Methylenchlorid aufgenommen und erneut filtriert. Das Filtrat enthält 2-Trifluormethoxy-benzylsulfonylisocyant als Rohware im Gemisch mit DABCO und wird als solches für die Folgeumsetzung weiterverwendet, da bei der Destillation im Hochvakuum teilweise Zersetzung eintritt.

Nach dem in Beispiel (II-1) exemplarisch beschriebenen Verfahren können die in der nachstehenden Tabelle 3 aufgeführten Verbindungen der Formel (II) hergestellt werden:

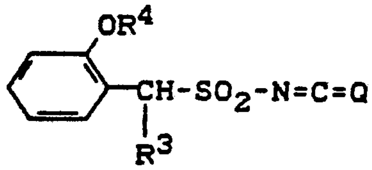 (II)

Tabelle 3

| Beispiel Nr. | $R^3$ | $R^4$ | Q | Physikalische Konstante |
|---|---|---|---|---|
| (II-2) | H | $CHF_2$ | O | |
| (II-3) | H | $CF_2CHF_2$ | O | |
| (II-4) | H | $CF_2CHFCl$ | O | |
| (II-5) | $CH_3$ | $CF_3$ | O | |
| (II-6) | $CH_3$ | $CHF_2$ | O | |
| (II-7) | H | $CH_2-CF_3$ | O | |
| (II-8) | H | $CH_2CH_2Cl$ | O | |
| (II-9) | $CH_3$ | $CH_2-CF_3$ | O | |
| (II-10) | $CH_3$ | $CH_2CH_2Cl$ | O | |
| (II-11) | H | $CClF_2$ | O | |
| (II-12) | H | $CCl_2F$ | O | |

Zwischenprodukte der Formel (IV)

Beispiel (IV-1)

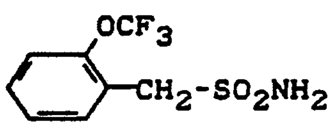

205,9 g (0,75 Mol) 2-Trifluormethoxy-benzylsulfochlorid werden bei 30 °C - 40 °C in 1,5 l gesättigte wässrige Ammoniaklösung eingetragen und 3 Stunden bei 50 °C -60 °C nachgerührt. Nach dem Abkühlen wird der ausgefallene Niederschlag abgesaugt, mit Wasser neutral gewaschen und getrocknet.

Man erhält 136,5 g (71 % der Theorie) 2-Trifluormethoxybenzylsulfonsäureamid vom Schmelzpunkt 127 °C.

Nach dem in Beispiel (IV-1) exemplarisch beschriebenen Verfahren können die in der nachstehenden Tabelle 4 aufgeführten Verbindungen der Formel (IV) hergestellt werden:

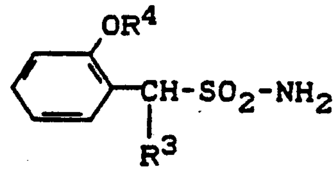

(IV)

Tabelle 4

| Beispiel-Nr. | $R^3$ | $R^4$ | Schmelzpunkt [°C] |
|---|---|---|---|
| (IV-2) | H | $CHF_2$ | |
| (IV-3) | H | $CF_2CHF_2$ | |
| (IV-4) | H | $CF_2CHFCl$ | |
| (IV-5) | $CH_3$ | $CF_3$ | |
| (IV-6) | $CH_3$ | $CHF_2$ | |
| (IV-7) | H | $CH_2-CF_3$ | |
| (IV-8) | H | $CH_2CH_2Cl$ | |
| (IV-9) | $CH_3$ | $CH_2-CF_3$ | |
| (IV-10) | $CH_3$ | $CH_2CH_2Cl$ | |
| (IV-11) | H | $CClF_2$ | |
| (IV-12) | H | $CCl_2F$ | |

Zwischenprodukte der Formel (IX)

Beispiel (IX-1)

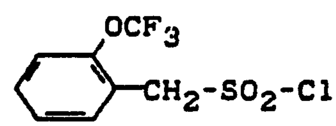

23,7 g (0,085 Mol)2-Trifluormethoxy-benzylsulfonsäure-Natriumsalz werden mit 35,5 g (0,17 Mol) Phosphorpentachlorid vermischt und ca. 2 Stunden bei 80 °C - 90 °C Badtemperatur am Rotationsverdampfer umgeschwenkt. Es wird abgekühlt und das gebildete Phosphoroxychlorid im Vakuum entfernt. Der Rückstand wird in Methylenchlorid suspendiert und auf Eiswasser gegossen. Die organische Phase wird abgetrennt, neutral gewaschen, getrocknet und eingeengt.

Man erhält 19,0 g (81,4 % der Theorie) 2-Trifluormethoxy-benzylsulfonsäurechlorid als Rohware, das für die folgende Umsetzung zum Sulfonamid eine hinreichende Reinheit aufweist. Zur Reinigung kann die Rohware in Methylenchlorid aufgenommen und über Kieselgel gereinigt werden: $n_D^{22,5}$ = 1,4854.

Nach dem in Beispiel (IX-1) exemplarisch beschriebenen Verfahren können die in der nachstehenden Tabelle 5 aufgeführten Verbindungen der Formel (IX) hergestellt werden:

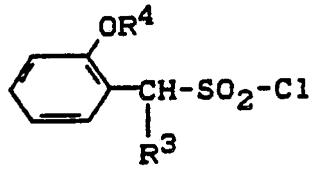

(IX)

Tabelle 5

| Beispiel-Nr. | $R^3$ | $R^4$ | Physikalische Konstante |
|---|---|---|---|
| (IX-2) | H | $CHF_2$ | |
| (IX-3) | H | $CF_2CHF_2$ | |
| (IX-4) | H | $CF_2CHFCl$ | |
| (IX-5) | $CH_3$ | $CF_3$ | |
| (IX-6) | $CH_3$ | $CHF_2$ | |
| (IX-7) | H | $CH_2-CF_3$ | |
| (IX-8) | H | $CH_2CH_2Cl$ | |
| (IX-9) | $CH_3$ | $CH_2-CF_3$ | |
| (IX-10) | $CH_3$ | $CH_2CH_2Cl$ | |
| (IX-11) | H | $CClF_2$ | |
| (IX-12) | H | $CCl_2F$ | |

Zwischenprodukte der Formel (X)

Beispiel (X-1)

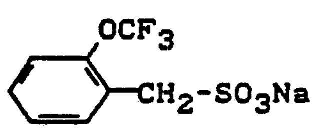

21,0 g (0,1 Mol) 2-Trifluormethoxy-benzylchlorid werden mit einer gesättigten Lösung aus 13,9 g (0,11 Mol) Natriumthiosulfat in Wasser unter gutem Rühren 5 Stunden unter Rückfluß erhitzt. Nach dem Abkühlen wird der ausgefallene weiße Niederschlag abgesaugt und mit wenig eiskaltem Wasser nachgewaschen.

Nach Trocknen über Phorphorpentoxid werden 26,4 g (95 % der Theorie) 2-Trifluormethoxy-benzylsulfonsäure-Natriumsalz vom Schmelzpunkt 115 °C erhalten.

Nach dem in Beispiel (X-1) exemplarisch beschriebenen Verfahren können die in der nachstehenden Tabelle 6 aufgeführten Verbindungen der Formel (X) hergestellt werden:

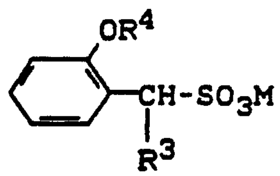

(X)

**Tabelle 6**

| Beispiel-Nr. | $R^3$ | $R^4$ | M | Schmelzpunkt [°C] |
|---|---|---|---|---|
| (X-2) | H | $CHF_2$ | Na | |
| (X-3) | H | $CF_2CHF_2$ | Na | |
| (X-4) | H | $CF_2CHFCl$ | Na | |
| (X-5) | $CH_3$ | $CF_3$ | Na | |
| (X-6) | $CH_3$ | $CHF_2$ | Na | |
| (X-7) | H | $CH_2-CF_3$ | Na | |
| (X-8) | H | $CH_2CH_2Cl$ | Na | |
| (X-9) | $CH_3$ | $CH_2-CF_3$ | Na | |
| (X-10) | $CH_3$ | $CH_2CH_2Cl$ | Na | |
| (X-11) | H | $CClF_2$ | Na | |
| (X-12) | H | $CCl_2F$ | Na | |
| (X-13) | H | $CF_3$ | K | |

Zwischenprodukte der Formel (XI)

Beispiel (XI-1)

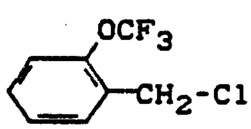

900 g (5,1 Mol) 2-Trifluormethoxy-toluol (2-Methyl-trifluoranisol) werden auf 100 °C erhitzt und bei dieser Temperatur werden unter UV-Bestrahlung 180 g (2,54 Mol) Chlor eingeleitet. Dann wird Stickstoff durchgeblasen und das Reaktionsgemisch wird unter vermindertem Druck fraktioniert destilliert.

Als Hauptfraktion erhält man 425 g (40 % der Theorie) 2-Trifluormethoxy-benzylchlorid (2-Chlormethyl-trifluoranisol) vom Siedepunkt 110 °C/100 mbar und vom Brechungsindex $n_D^{20}$ = 1,5450.

Nach dem in Beispiel (XI-1) exemplarisch beschriebenen Verfahren können die in der nachstehenden Tabelle 7 aufgeführten Verbindungen der Formel (XI) hergestellt werden:

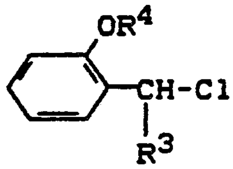

(XI)

**Tabelle 7**

| Beispiel-Nr. | $R^3$ | $R^4$ | Brechungsindex |
|---|---|---|---|
| (XI-2) | H | $CHF_2$ | |
| (XI-3) | H | $CF_2CHF_2$ | |
| (XI-4) | H | $CF_2CHFCl$ | |
| (XI-5) | $CH_3$ | $CF_3$ | |
| (XI-6) | $CH_3$ | $CHF_2$ | |
| (XI-7) | H | $CH_2-CF_3$ | |
| (XI-8) | H | $CH_2CH_2Cl$ | |
| (XI-9) | $CH_3$ | $CH_2-CF_3$ | |
| (XI-10) | H | $CF_2Cl$ | |
| (XI-11) | H | $CFCl_2$ | |

Anwendungsbeispiele

In den folgenden Anwendungsbeispielen wird die nachstehend aufgeführte Verbindung als Vergleichssubstanz herangezogen:

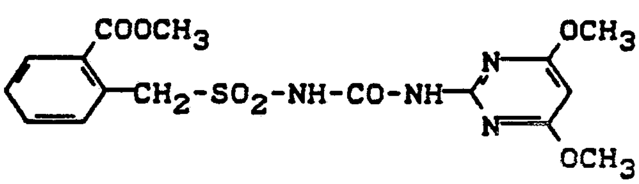

(A)

1-(2-Methoxycarbonyl-benzylsulfonyl)-3-(4,6-dimethoxypyrimidin-2-yl)-harnstoff (bekannt aus US-PS 4 420 325).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelt Kontrolle)
100 % = totale Vernichtung

Die erfindungsgemäßen Wirkstoffe zeigen in diesem Test eine sehr gute herbizide Wirksamkeit. Beispielsweise zeigt in diesem Test der Wirkstoff gemäß Herstellungsbeispiel (2) eine bessere Verträglichkeit gegenüber Kulturpflanzen, wie z. B. Reis und Mais, und eine bessere Wirkung gegen Problemunkräuter, wie z. B. Galium und Ipomoea, als die Vergleichsverbindung (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 200 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Die erfindungsgemäße Wirkstoffe zeigen in diesem Test eine sehr gute herbizide Wirksamkeit. Beispielweise zeigt in diesem Test der Wirkstoff gemäß Herstellungsbeispiel (2) eine bessere Verträglichkeit gegenüber Kulturpflanzen, wie z. B. Mais, und eine bessere Wirkung gegen Problemunkräuter, wie z. B. Amaranthus, Datura, Sinapis und Xanthium, als die Vergleichsverbindung (A).

## Ansprüche

1) Substituierte 1-Benzylsulfonyl-3-heteroaryl-(thio)harnstoffe der allgemeinen Formel (I)

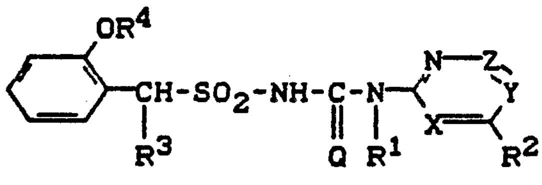

(I)

in welcher
Q für Sauerstoff oder Schwefel steht,

$R^1$ für Wasserstoff oder für gegebenenfalls substituierte Reste aus der Reihe Alkyl, Alkenyl, Alkinyl oder Aralkyl steht,
$R^2$ für Wasserstoff, Halogen, Hydroxy, Alkyl, Halogenalkyl, Alkoxy, Halogenalkoxy, Alkylthio, Halogenalkylthio, Amino, Alkylamino oder Dialkylamino steht,
$R^3$ für Wasserstoff oder Alkyl steht,
$R^4$ für Halogenalkyl steht,
X für Stickstoff oder eine -CH-Gruppierung steht,
Y für Stickstoff oder eine -$CR^5$-Gruppierung steht, worin
$R^5$ für Wasserstoff, Halogen, Cyano, Alkyl, Formyl, Alkyl-carbonyl oder Alkoxy-carbonyl steht und
Z für Stickstoff oder eine -$CR^6$-Gruppierung steht, worin
$R^6$ für Wasserstoff, Halogen, Hydroxy, Alkyl, Halogenalkyl, Alkoxy, Halogenalkoxy, Alkylthio, Alkylamino oder Dialkylamino steht,
sowie Salze von Verbindungen der Formel (I) mit Metallen und basischen organische Stickstoffverbindungen.

2) Substitutiert 1-Benzylsulfonyl-3-heteroaryl-(thio)harnstoffe der allgemeinen Formel (I) gemäß Anspruch 1, dadurch gekennzeichnet, daß darin
Q für Sauerstoff oder Schwefel steht,
$R^1$ für Wasserstoff oder für $C_1$-$C_6$-Alkyl [welches gegebenenfalls durch Fluor, Cyano, $C_1$-$C_4$-Alkoxy oder $C_1$-$C_4$-Alkylthio substituiert ist], für $C_3$-$C_6$-Alkenyl und $C_3$-$C_6$-Alkinyl [welche gegebenenfalls durch Fluor oder Chlor substituiert sind] oder für Phenyl-$C_1$-$C_2$-alkyl [welches im Phenylteil gegebenenfalls durch Fluor, Chlor, Nitro, Cyano, Methyl, Methoxy oder $C_1$-$C_2$-Alkoxycarbonyl substituiert ist] steht,
$R^2$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethylamino, Dimethylamino oder Diethylamino steht,
$R^3$ für Wasserstoff oder $C_1$-$C_4$-Alkyl steht,
$R^4$ für Halogen-$C_1$-$C_4$-alkyl steht, wobei Halogen für Fluor und/oder Chlor steht,
X für Stickstoff oder eine -CH-Gruppierung steht,
Y für Stickstoff oder eine -$CR^5$-Gruppierung steht, worin
$R^5$ für Wasserstoff, Fluor, Chlor, Brom, Methyl, Formyl, Acetyl, Methoxycarbonyl oder Ethoxycarbonyl steht und
Z für Stickstoff oder eine -$CR^6$-Gruppierung steht, worin
$R^6$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

3) Verfahren zur Herstellung von substituierten 1-Benzylsulfonyl-3-heteroaryl-(thio)harnstoffen der allgemeinen Formel (I) gemäß Anspruch 1, dadurch gekennzeichnet, daß man 2-Halogenalkoxy-benzylsulfonyl-iso(thio)cyanate der Formel (II)

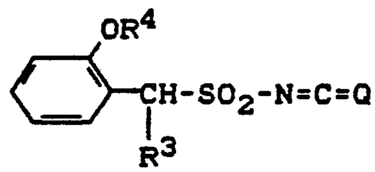

(II)

in welcher
Q, $R^3$ und $R^4$ die in Anspruch 1 angegebenen Bedeutungen haben,
mit Heteroarylamine in Formel (III)

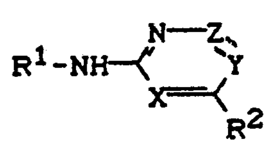

(III)

in welcher
$R^1$, $R^2$, X, Y und Z die in Anspruch 1 angegebenen Bedeutungen haben,
gegebenenfalls in Gegenwart von Verdünnungsmitteln und gegebenenfalls in Gegenwart von Katalysatoren umsetzt und gegebenenfalls die so erhaltenen Produkte nach üblichen Methoden in Salze überführt.

4) Herbizide Mittel, gekennzeichnet durch einen Gehalt an mindestens einem substituierten 1-Benzylsulfonyl-3-heteroaryl-(thio)harnstoff der allgemeinen Formel (I) gemäß Anspruch 1.

5) Verwendung von substituierten 1-Benzylsulfonyl-3-heteroaryl-(thio)harnstoffen der allgemeinen Formel (I) gemäß Anspruch 1 zur Bekämpfung von unerwünschtem Pflanzenwachstum.

6) Verfahren zur Herstellung von herbiziden Mitteln, dadurch gekennzeichnet, daß man 1-Benzylsulfonyl-3-heteroaryl-(thio)harnstoffe der allgemeinen Formel (I) gemäß Anspruch 1 mit Streckmitteln und/oder oberflächenaktiven Mitteln vermischt.

7) 2-Halogenalkoxy-benzylsulfonyl-iso(thio)cyanate der allgemeinen Formel (II)

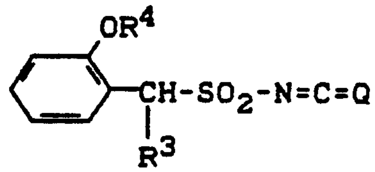

(II)

in welcher
Q für Sauerstoff oder Schwefel steht,
$R^3$ für Wasserstoff oder Alkyl steht und
$R^4$ für Halogenalkyl steht.

8) Verfahren zur Herstellung von 2-Halogenalkoxy-benzylsulfonyl-iso(thio)cyanaten der allgemeinen Formel (II) gemäß Anspruch 7, dadurch gekennzeichnet, daß man

(a) für den Fall, daß Q für Sauerstoff steht, 2-Halogenalkoxy-benzylsulfonamide der Formel (IV)

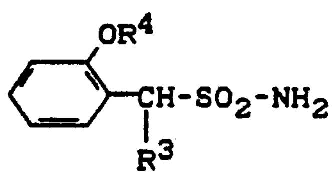

(IV)

in welcher
$R^3$ und $R^4$ die in Anspruch 1 angegebenen Bedeutungen haben,
mit etwa der äquimolaren Menge eines Alkylisocyanats gegebenenfalls in Gegenwart eines Katalysators und in Gegenwart eines Verdünnungsmittels bei Temperaturen zwischen 50 °C und 200 °C umsetzt und dabei mindestens die äquimolare Menge Phosgen einleitet,
oder

(b) für den Fall, daß Q für Schwefel steht, daß man 2-Halogenalkoxy-benzylsulfonamide der obigen Formel (IV) mit Schwefelkohlenstoff in Gegenwart eines Säureakzeptors und gegebenen falls in Gegenwart eines Verdünnungsmittels bei Temperaturen zwischen 0 °C und 50 °C umsetzt und anschließend mit Phosgen oder Thionylchlorid, gegebenenfalls in Gegenwart eines Verdünnungsmittels bei Temperaturen zwischen 0 °C und 50 °C umsetzt.

9) 2-Halogenalkoxy-benzylsulfonamide der allgemeinen Formel (IV)

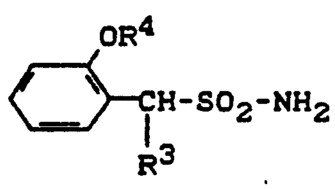

(IV)

in welcher
$R^3$ für Wasserstoff oder Alkyl steht und
$R^4$ für Halogenalkyl steht.

10) Verfahren zur Herstellung von 2-Halogenalkoxy-benzylsulfonamiden der allgemeinen Formel (IV) gemäß Anspruch 9, dadurch gekennzeichnet, daß man 2-Halogenalkoxy-benzylsulfonsäurechloride der Formel (IX)

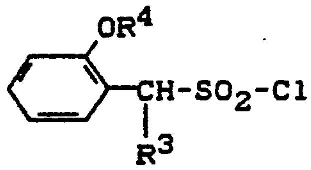

(IX)

in welcher

$R^3$ und $R^4$ die in Anspruch 1 angegebenen Bedeutungen haben,

mit Ammoniak, gegebenenfalls in Gegenwart eines Verdünnungsmittels, bei Temperaturen zwischen -20 °C und +100 °C umsetzt.

11) 2-Halogenalkoxy-benzylsulfonsäurechloride der allgemeinen Formel (IX)

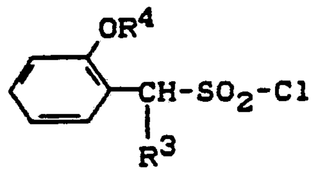

(IX)

in welcher

$R^3$ für Wasserstoff oder Alkyl steht und

$R^4$ für Halogenalkyl steht.

12) Verfahren zur Herstellung von 2-Halogenalkoxy-benzylsulfonsäurechloride der allgemeinen Formel (IX) gemäß Anspruch 11, dadurch gekennzeichnet, daß man 2-Halogenalkoxy-benzylsulfonsäurechloride bzw. deren Salze der Formel (X)

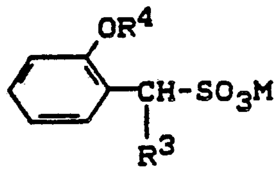

(X)

in welcher

M für Wasserstoff oder ein Alkalimetall steht und

$R^3$ und $R^4$ die in Anspruch 1 angegebenen Bedeutungen haben,

mit wenigstens der äquimolaren Menge eines Chlorierungsmittels, gegebenenfalls in Gegenwart eines Verdünnungsmittels, bei Temperaturen zwischen 0 °C und 150 °C umsetzt.

13) 2-Halogenalkoxy-benzylsulfonsäurechloride bzw. deren Salze der allgemeinen Formel (X)

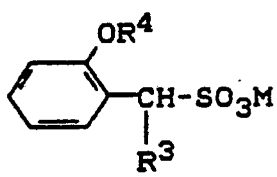

(X)

in welcher

M für Wasserstoff oder ein Alkalimetall steht,

$R^3$ für Wasserstoff oder Alkyl steht und

$R^4$ für Halogenalkyl steht,

14) Verfahren zur Herstellung von 2-Halogenalkoxy-benzylsulfonsäurechloride bzw. deren Salze der allgemei nen Formel (X) gemäß Anspruch 13, dadurch gekennzeichnet, daß man 2-Halogenalkoxy-benzylchloride der Formel (XI)

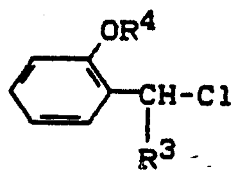

(XI)

in welcher

$R^3$ und $R^4$ die in Anspruch 1 angegebenen Bedeutungen haben, mit wenigstens der äquimolaren Menge Natrium-bzw. Kaliumthiosulfat in Wasser bei Temperaturen zwischen 20 °C und 110 °C umsetzt und nach üblichen Methode aufarbeitet.

15) 2-Trifluormethoxy-benzylchlorid der nachstehenden Formel

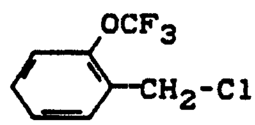

16) Verfahren zur Herstellung von 2-Trifluormethoxybenzylchlorid, dadurch gekennzeichnet, daß man 2-Trifluormethoxytoluol der nachstehenden Formel

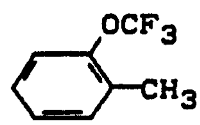

mit höchstens der äquimolaren Menge Chlor bei Temperaturen zwischen 50 °C und 180 °C unter UV-Bestrahlung umsetzt und nach üblichen Methoden aufarbeitet.